(19) Europäisches Patentamt
European Patent Office
Office européen des brevets

(11) EP 4 299 573 A1

(12) **EUROPEAN PATENT APPLICATION**

(43) Date of publication:
**03.01.2024 Bulletin 2024/01**

(21) Application number: **22182147.3**

(22) Date of filing: **30.06.2022**

(51) International Patent Classification (IPC):
***C07D 403/10*** *(2006.01)* ***H10K 85/00*** *(2023.01)*

(52) Cooperative Patent Classification (CPC):
**C07D 403/10; H10K 85/615; H10K 85/654;**
H10K 50/18

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novaled GmbH**
**01099 Dresden (DE)**

(72) Inventors:
- **KARPOV, Yevhen**
  **01099 Dresden (DE)**
- **PAVICIC, Domagoj**
  **01099 Dresden (DE)**
- **GALÁN GARCÍA, Elena**
  **01099 Dresden (DE)**

(74) Representative: **Bittner, Thomas L.**
**Boehmert & Boehmert**
**Anwaltspartnerschaft mbB**
**Pettenkoferstrasse 22**
**80336 München (DE)**

(54) **COMPOUND, ORGANIC SEMICONDUCTING MATERIAL COMPRISING THE SAME, ORGANIC ELECTRONIC DEVICE COMPRISING THE SAME AND A METHOD FOR PREPARING THE ORGANIC ELECTRONIC DEVICE**

(57) The present invention relates to a compound, an organic semiconducting material comprising the same, an organic electronic device comprising the same and a method for preparing the organic electronic device.

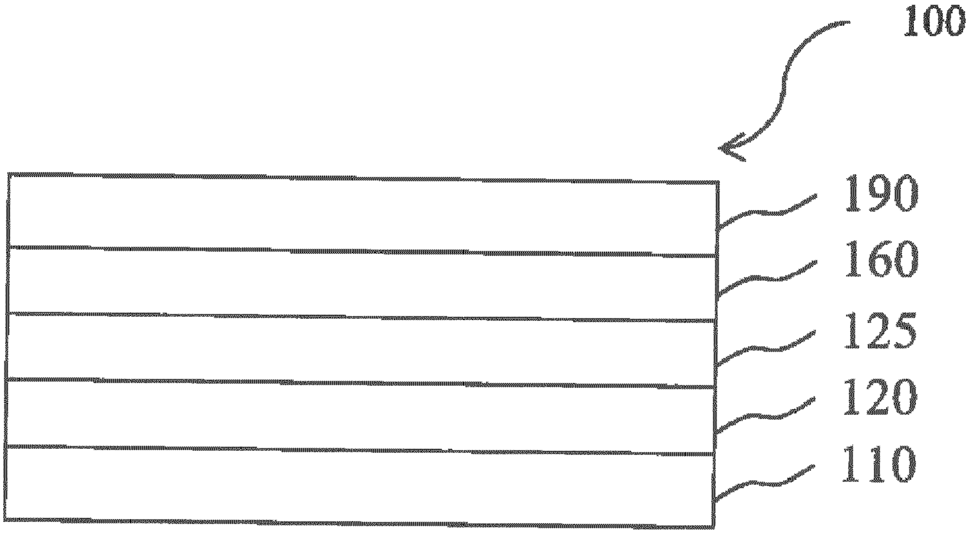


Fig.1

EP 4 299 573 A1

**Description**

TECHNICAL FIELD

The present invention relates to a compound, an organic semiconducting material comprising the same, an organic electronic device comprising the same and a method for preparing the organic electronic device.

BACKROUND OF THE INVENTION

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the organic semiconductor layer, and among them, may be affected by characteristics of an organic material of the organic semiconductor layer.

Particularly, development of an organic semiconductor layer being capable of increasing electron mobility and simultaneously increasing electrochemical stability is needed so that the organic electronic device, such as an organic light emitting diode, may be applied to a large-size flat panel display.

Further, development of an organic semiconductor layer being capable to have an extended life span at higher current density and thereby at higher brightness is needed. In particular the development of an organic semiconductor material or semiconductor layer is needed with respect to lowering the operating voltage, which is important for reducing power consumption and increasing battery life, for example of a mobile display device.

There remains a need to improve performance of organic semiconductor materials, organic semiconductor layers, as well as organic electronic devices thereof, in particular to achieve increased efficiency through improving the characteristics of the compounds comprised therein.

It is, therefore, the object of the present invention to provide an organic light emitting diode overcoming drawbacks of the prior art, in particular providing compounds for use in organic semiconducting materials and electronic devices comprising the same helpful to improve driving voltage and/or efficiency thereof.

DISCLOSURE

This object is achieved by a compound of the following formula (I)

$$A\text{-}(Cy)_n\text{-}Pz\text{-}FL \qquad (I)$$

wherein

- A is selected from substituted or unsubstituted $C_{10}$ to $C_{42}$ aryl comprising at least two condensed aromatic rings and from $C_2$-$C_{42}$ heteroaryl, wherein it is excluded that the $C_2$ to $C_{42}$ heteroaryl is benzoxazolyl or carbazolyl;

  - wherein, if A is substituted, the substituent(s) is/are independently selected from the group consisting of $C_6$-$C_{18}$ aryl, $C_3$-$C_{20}$ heteroaryl, D, F, CN, $C_1$-$C_{20}$ alkyl, $C_1$-$C_{20}$ alkoxy, $PY(R)_2$, OR, SR, (C=O)R, (C=O)N(R)$_2$, Si(R)$_3$, (S=O)R, and

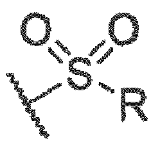,

whereby

Y is O or S, and

R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;

- Cy is independently selected from a substituted or unsubstituted monocyclic $C_3$ to $C_{36}$ hydrocarbon group, a substituted or unsubstituted polycyclic $C_3$ to $C_{36}$ hydrocarbon group, a substituted or unsubstituted monocyclic heteroatom-containing $C_3$ to $C_{36}$ hydrocarbon group, or a substituted or unsubstituted polycyclic heteroatom-containing $C_3$ to $C_{36}$ hydrocarbon group;

  - wherein, if Cy is substituted, the substituent(s) is/are independently selected from the groups consisting of $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, $(C=O)N(R)_2$, $Si(R)_3$, (S=O)R, and

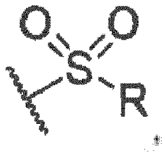

  whereby

  Y is O or S, and

  R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;

- n is an integer from 0 to 4;

- Pz is fully substituted pyrazinylene;

  - wherein the substituents on the pyrazinylene are independently selected from groups having at least one carbon atom;

- FL is substituted or unsubstituted fluorenyl;

  - wherein, if FL is substituted, the substituent(s) is/are independently selected from the groups consisting of $C_6$-$C_{18}$ aryl, $C_2$-$C_{20}$ heteroaryl, D, F, CN, $C_1$-$C_{16}$ alkyl, $C_1$-$C_{16}$ alkoxy, $PY(R)_2$, OR, SR, (C=O)R, $(C=O)N(R)_2$, $Si(R)_3$, (S=O)R, and

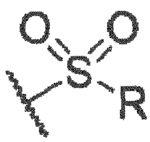

,

  whereby

  Y is O or S, and
  R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl.

The object is further achieved by an organic electronic semiconducting material comprising the compound in accordance with the invention.

The object is further achieved by an organic electronic device comprising a semiconducting layer, wherein the

semiconducting layer comprises the compound according to the invention.

The object is further achieved by a process for preparing an organic electronic device according to the invention comprising a step of depositing the compound according to the invention on a solid support.

Surprisingly, it was found that the organic electronic device according to the invention solves the problem underlying the present invention by enabling devices in various aspects superior over the organic electroluminescent devices known in the art, in particular with respect to efficiency and/or operating voltage.

The invention is especially related to a compound of the following formula (I)

$$A\text{-}(Cy)_n\text{-}Pz\text{-}FL \qquad (I).$$

A is selected from substituted or unsubstituted $C_{10}$ to $C_{42}$ aryl comprising at least two condensed aromatic rings and from $C_2$-$C_{42}$ heteroaryl. In accordance with the invention, it is excluded that, in case that A is $C_2$-$C_{42}$ heteroaryl, the $C_2$-$C_{42}$ heteroaryl is benzoxazolyl or carbazolyl. It is also provided that A does not comprise benzoxazolyl or carbazolyl. It may further be provided that the compound of Formula (I) comprises benzoxazolyl or carbazolyl. Avoiding the presence of benzoxazolyl or carbazolyl in the compound of formula (I), especially in the moiety A, was found to be advantageous in view of the device performance.

A may be selected from substituted or unsubstituted $C_{10}$ to $C_{36}$ aryl comprising at least two condensed aromatic rings and from $C_2$-$C_{36}$ heteroaryl, wherein it is excluded that the $C_2$ to $C_{36}$ heteroaryl is benzoxazolyl or carbazolyl. A may be selected from substituted or unsubstituted $C_{10}$ to $C_{30}$ aryl comprising at least two condensed aromatic rings and from $C_3$-$C_{30}$ heteroaryl, wherein it is excluded that the $C_2$ to $C_{30}$ heteroaryl is benzoxazolyl or carbazolyl. A may be selected from substituted or unsubstituted $C_{10}$ to $C_{24}$ aryl comprising at least two condensed aromatic rings and from $C_3$-$C_{24}$ heteroaryl, wherein it is excluded that the $C_2$ to $C_{24}$ heteroaryl is benzoxazolyl or carbazolyl. A may be selected from substituted or unsubstituted $C_{10}$ to $C_{18}$ aryl comprising at least two condensed aromatic rings and from $C_3$-$C_{21}$ heteroaryl, wherein it is excluded that the $C_2$ to $C_{21}$ heteroaryl is benzoxazolyl or carbazolyl.

A comprises at least one six-membered N-containing aromatic ring.

A may be selected from $C_2$-$C_{36}$ heteroaryl, wherein it is excluded that the $C_2$ to $C_{36}$ heteroaryl is benzoxazolyl or carbazolyl. A may be selected from $C_3$-$C_{30}$ heteroaryl, wherein it is excluded that the $C_2$ to $C_{30}$ heteroaryl is benzoxazolyl or carbazolyl. A may be selected from $C_3$-$C_{24}$ heteroaryl, wherein it is excluded that the $C_2$ to $C_{24}$ heteroaryl is benzoxazolyl or carbazolyl. A may be selected from $C_3$-$C_{21}$ heteroaryl, wherein it is excluded that the $C_2$ to $C_{21}$ heteroaryl is benzoxazolyl or carbazolyl.

A is selected from the group consisting of aryl comprising 2, 3, 4 or 5 condensed aromatic rings, azinyl, diazinyl, triazinyl, azanaphtalenyl, diazanaphtalenyl, triazanaphtalenyl, azaanthracenyl, azaphenanthrenyl, diazaanthracenyl, diazaphenanthrenyl, triazaanthracenyl, triazaphenanthrenyl, benzofuranyl and aza-, diaza- or triaza- derivatives thereof, benzothiophenyl and aza-, diaza- or triaza- derivatives thereof, dibenzofuranyl and aza-, diaza- or triaza- derivatives thereof, dibenzothiophenyl and aza-, diaza- or triaza- derivatives thereof, naphtofuranyl and aza-, diaza- or triaza- derivatives thereof, naphtothiophenyl and aza-, diaza- or triaza- derivatives thereof, naphtobenzofuranyl and aza-, diaza- or triaza- derivatives thereof, naphtobenzothiophenyl and aza-, diaza- or triaza- derivatives thereof, dinaphtofuranyl and aza-, diaza- or triaza- derivatives thereof, and dinaphtothiophenyl and aza-, diaza- or triaza- derivatives thereof, which are substituted or unsubstituted, respectively.

The term "condensed aryl rings" is understood the way that two aryl rings are considered fused or condensed when they share at least two common $sp^2$-hybridized carbon atoms.

The term "aza-derivative" refers to a derivative of a specific moiety in which one carbon atom is replaced by a nitrogen atom. The term "diaza-derivative" refers to a derivative of a specific moiety in which two carbon atom are replaced by two nitrogen atoms, respectively. The term "triaza-derivative" refers to a derivative of a specific moiety in which three carbon atom are replaced by three nitrogen atoms, respectively.

A may be selected from the group consisting of azinyl, diazinyl, triazinyl, azanaphtalenyl, diazanaphtalenyl, triazanaphtalenyl, azaanthracenyl, azaphenanthrenyl, diazaanthracenyl, diazaphenanthrenyl, triazaanthracenyl, triazaphenanthrenyl, benzofuranyl and aza-, diaza- or triaza- derivatives thereof, benzothiophenyl and aza-, diaza- or triaza-derivatives thereof, dibenzofuranyl and aza-, diaza- or triaza- derivatives thereof, dibenzothiophenyl and aza-, diaza- or triaza- derivatives thereof, naphtofuranyl and aza-, diaza- or triaza- derivatives thereof, naphtothiophenyl and aza-, diaza- or triaza- derivatives thereof, naphtobenzofuranyl and aza-, diaza- or triaza- derivatives thereof, naphtobenzothiophenyl and aza-, diaza- or triaza- derivatives thereof, dinaphtofuranyl and aza-, diaza- or triaza- derivatives thereof, and dinaphtothiophenyl and aza-, diaza- or triaza- derivatives thereof, which are substituted or unsubstituted, respectively.

A may be selected from naphtalenyl, anthracenyl, phenanthrenyl, triphenylenyl, acenaphtenyl, fluoranthenyl, pyrenyl, tetracenyl, pyridinyl, pyrazinyl, pyrimidinyl, pyridazinyl, triazinyl, quinolinyl, isoquinolinyl, cinnolinyl, phtalazinyl, quinazolinyl, quinoxalinyl, naphtyridinyl, imidazopyridinyl, phenathridinyl, phenanthrolinyl, acridinyl, benzoacridinyl, dibenzoacridinyl, which are substituted or unsubstituted, respectively.

A may be selected from pyridinyl, pyrazinyl, pyrimidinyl, pyridazinyl, triazinyl, quinolinyl, isoquinolinyl, cinnolinyl,

phtalazinyl, quinazolinyl, quinoxalinyl, naphtyridinyl, imidazopyridinyl, phenathridinyl, phenanthrolinyl, acridinyl, benzoacridinyl, dibenzoacridinyl, which are substituted or unsubstituted, respectively.

A may be selected from pyridinyl, pyrazinyl, pyrimidinyl, pyridazinyl or triazinyl. A may be selected from pyrimidinyl or triazinyl.

If A is substituted, the substituent(s) is/are independently selected from the group consisting of $C_6$-$C_{18}$ aryl, $C_3$-$C_{20}$ heteroaryl, D, F, CN, $C_1$-$C_{20}$ alkyl, $C_1$-$C_{20}$ alkoxy, $PY(R)_2$, OR, SR, (C=O)R, (C=O)N(R)$_2$, Si(R)$_3$, (S=O)R, and

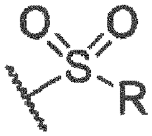,

whereby

Y is O or S, and

R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$) aryl and $C_3$-$C_{20}$ heteroaryl. In case that the one or more substituents on A comprise more than one group R, the respective R can be selected independently from each other, that is, may independently be the same or different from each other.

If A is substituted, the substituent(s) may independently be selected from the group consisting of $C_6$-$C_{18}$ aryl, $C_3$-$C_{20}$ heteroaryl, D, F, CN, $C_1$-$C_{20}$ alkyl. If A is substituted, the substituent(s) may independently be selected from the group consisting of $C_6$-$C_{18}$ aryl and $C_3$-$C_{20}$ heteroaryl. If A is substituted, the substituent(s) may independently be selected from the group consisting of $C_6$-$C_{18}$ aryl. If A is substituted, the substituent(s) may independently be selected from the group consisting of $C_6$-$C_{12}$ aryl. If A is substituted, the substituent(s) may be phenyl, respectively.

Cy is independently selected from a substituted or unsubstituted monocyclic $C_3$ to $C_{36}$ hydrocarbon group, a substituted or unsubstituted polycyclic $C_3$ to $C_{36}$ hydrocarbon group, a substituted or unsubstituted monocyclic heteroatom-containing $C_3$ to $C_{36}$ hydrocarbon group, or a substituted or unsubstituted polycyclic heteroatom-containing $C_3$ to $C_{36}$ hydrocarbon group. Cy may independently be selected from a substituted or unsubstituted monocyclic $C_6$ to $C_{30}$ hydrocarbon group, a substituted or unsubstituted polycyclic $C_6$ to $C_{30}$ hydrocarbon group, a substituted or unsubstituted monocyclic heteroatom-containing $C_3$ to $C_{30}$ hydrocarbon group, or a substituted or unsubstituted polycyclic heteroatom-containing $C_3$ to $C_{30}$ hydrocarbon group. Cy may independently be selected from a substituted or unsubstituted monocyclic $C_6$ to $C_{24}$ hydrocarbon group, a substituted or unsubstituted polycyclic $C_6$ to $C_{24}$ hydrocarbon group, a substituted or unsubstituted monocyclic heteroatom-containing $C_3$ to $C_{24}$ hydrocarbon group, or a substituted or unsubstituted polycyclic heteroatom-containing $C_3$ to $C_{24}$ hydrocarbon group. Cy may independently be selected from a substituted or unsubstituted monocyclic $C_6$ to $C_{18}$ hydrocarbon group, a substituted or unsubstituted polycyclic $C_6$ to $C_{18}$ hydrocarbon group, a substituted or unsubstituted monocyclic heteroatom-containing $C_3$ to $C_{18}$ hydrocarbon group, or a substituted or unsubstituted polycyclic heteroatom-containing $C_3$ to $C_{18}$ hydrocarbon group. Cy may independently be selected from a substituted or unsubstituted monocyclic $C_6$ to $C_{12}$ hydrocarbon group, a substituted or unsubstituted polycyclic $C_6$ to $C_{12}$ hydrocarbon group, a substituted or unsubstituted monocyclic heteroatom-containing $C_3$ to $C_{12}$ hydrocarbon group, or a substituted or unsubstituted polycyclic heteroatom-containing $C_3$ to $C_{12}$ hydrocarbon group.

Cy may be independently selected from the group consisting of $C_6$-$C_{36}$ aryl and $C_3$-$C_{36}$ heteroaryl. Cy may be independently selected from the group consisting of $C_6$-$C_{30}$ aryl and $C_3$-$C_{30}$ heteroaryl. Cy may be independently selected from the group consisting of $C_6$-$C_{24}$ aryl and $C_3$-$C_{24}$ heteroaryl. Cy may be independently selected from the group consisting of $C_6$-$C_{12}$ aryl and $C_3$-$C_{12}$ heteroaryl.

Cy may be monocyclic or bicyclic, respectively. Cy may be monocyclic, respectively. Cy may independently be selected from phenylene and naphthenylene. Cy may be phenylene.

n is an integer from 0 to 4. n may be an integer from 1 to 4. n may be an integer from 1 to 3. n may be an integer from 1 to 2. n may be 1.

It may be provided that Cy is phenylene and n is 1. It may be provided that Cy is ortho-phenylene or meta-phenylene and n is 1. It may be provided that Cy is meta-phenylene and n is 1.

If Cy is substituted, the substituent(s) may independently be selected from the groups consisting of $C_6$-$C_{18}$ aryl, $C_3$-$C_{20}$ heteroaryl, D, F, CN, $C_1$-$C_{16}$ alkyl and $C_1$-$C_{16}$ alkoxy. If Cy is substituted, the substituent(s) may independently be selected from the groups consisting of D, F, CN, $C_1$-$C_{16}$ alkyl and $C_1$-$C_{16}$ alkoxy. If Cy is substituted, the substituent(s) may independently be selected from the groups consisting of D and $C_1$-$C_4$ alkyl. It may be provided that Cy is unsubstituted,

respectively.

Pz is fully substituted pyrazinylene. This means that the pyrazinylene is (1.) via one of the carbon atoms thereof connected to Cy (or to A in case that n is 0), (2.) via another carbon atoms thereof connected to FL, and (3.) two independently selected substituents thereof are bonded to the two remaining carbon atoms. Especially, the fully substituted pyrazinylene may have one of the following formulas

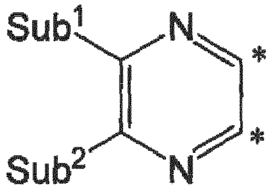 ; 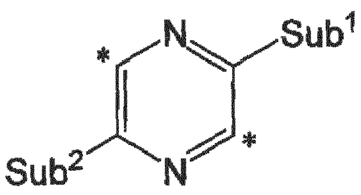 ; 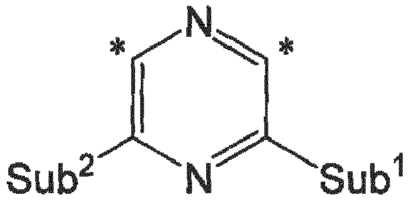

wherein "$Sub^1$" represents one substituent, "$Sub^2$" represents another substituent and "*" represent the binding positions to Cy (or A) and FL, respectively. It is not foreseen that two or more of the substituents of the fully substituted pyrazine are linked to each other to form a ring.

The substituents ($Sub^1$ and $Sub^2$) of the fully substituted pyrazinylene are independently selected from groups having at least one carbon atom. The substituents of the fully substituted pyrazinylene may independently be selected from a hydrocarbyl-group, a carbocyclic group, or a heterocyclic group. In one embodiment, the substituents of the fully substituted pyrazinylene are monocyclic or bicyclic.

The substituents of the fully substituted pyrazinylene may independently be selected from the group consisting of $C_6$-$C_{36}$ aryl and $C_3$-$C_{36}$ heteroaryl. The substituents of the fully substituted pyrazinylene may independently be selected from the group consisting of $C_6$-$C_{30}$ aryl and $C_3$-$C_{30}$ heteroaryl. The substituents of the fully substituted pyrazinylene may independently be selected from the group consisting of $C_6$-$C_{24}$ aryl and $C_3$-$C_{24}$ heteroaryl. The substituents of the fully substituted pyrazinylene may independently be selected from the group consisting of $C_6$-$C_{18}$ aryl and $C_3$-$C_{18}$ heteroaryl. The substituents of the fully substituted pyrazinylene may independently be selected from the group consisting of $C_6$-$C_{12}$ aryl and $C_3$-$C_{12}$ heteroaryl.

The substituents of the fully substituted pyrazinylene may independently be selected from the group consisting of $C_6$-$C_{36}$ aryl. The substituents of the fully substituted pyrazinylene may independently be selected from the group consisting of $C_6$-$C_{30}$ aryl. The substituents of the fully substituted pyrazinylene may independently be selected from the group consisting of $C_6$-$C_{24}$ aryl. The substituents of the fully substituted pyrazinylene may independently be selected from the group consisting of $C_6$-$C_{18}$ aryl. The substituents of the fully substituted pyrazinylene may independently be selected from the group consisting of $C_6$-$C_{12}$ aryl. The substituents of the fully substituted pyrazinylene may be phenyl, respectively.

FL may be substituted or unsubstituted fluorene-1-yl, substituted or unsubstituted fluorene-2-yl, substituted or unsubstituted fluorene-3-yl or substituted or unsubstituted fluorene-4-yl.

The fluorenyl may be substituted at the 9-position thereof. The fluorenyl may be substituted at the 9-position thereof with at least one hydrocarbyl group, especially with two hydrocarbyl groups which may be linked with each other to form a ring.

Reference is made herein to the following numbering of the fluorenyl moiety.

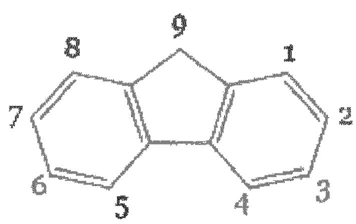

FL may be 9,9'-dialkyl-fluorenyl. FL may be 9,9'-$C_1$ to $C_{16}$-dialkyl-fluorenyl. FL may be 9,9'-$C_1$ to $C_{10}$-dialkyl-fluorenyl. FL may be 9,9'-$C_1$ to $C_4$-dialkyl-fluorenyl. FL may be 9,9'-dimethyl-fluorenyl.

The compound of formula (I) may be selected from the following E1 to E6.

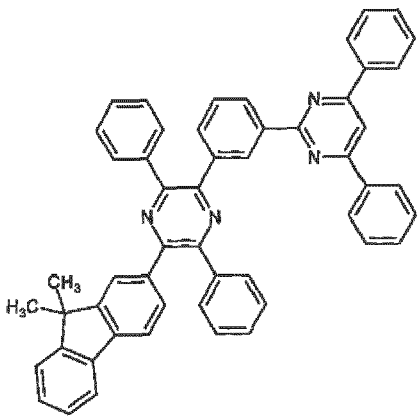

E1

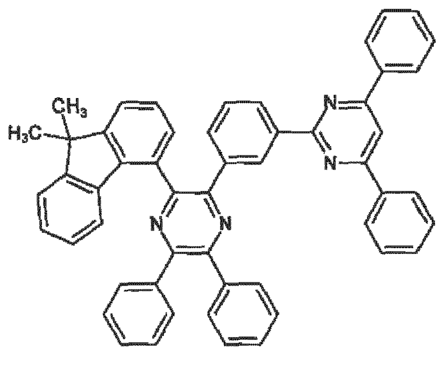

E2

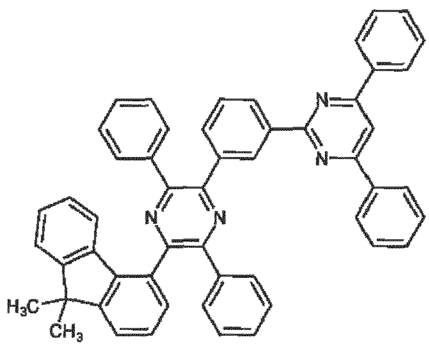

E3

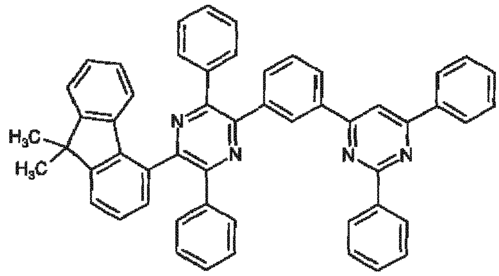

E4

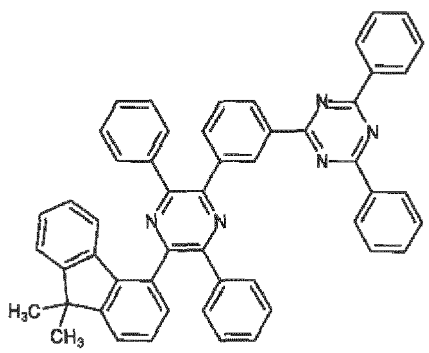

E5

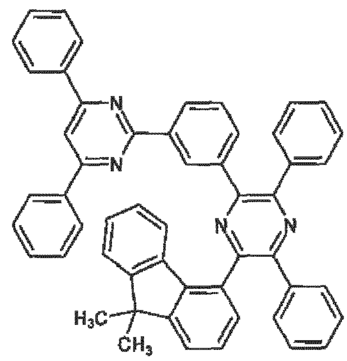

E6.

According to one embodiment, there is provided a compound of the following formula (I)

A-$(Cy)_n$-Pz-FL (I)

wherein

A is selected from $C_3$-$C_{30}$ heteroaryl, wherein it is excluded that the $C_2$ to $C_{30}$ heteroaryl is benzoxazolyl or carbazolyl; wherein, if A is substituted, the substituent(s) are independently selected from the group consisting of $C_6$-$C_{18}$ aryl, $C_3$-$C_{20}$ heteroaryl, D, F, CN, $C_1$-$C_{20}$ alkyl;

n is an integer from 1 to 4;

Cy is independently selected from the group consisting of $C_6$-$C_{36}$ aryl and $C_3$-$C_{36}$ heteroaryl; wherein, if Cy is substituted, the substituent(s) are independently selected from the groups consisting of D, F, CN, $C_1$-$C_{16}$ alkyl and $C_1$-$C_{16}$ alkoxy;

Pz is fully substituted pyrazinylene, wherein the substituents of the fully substituted pyrazinylene are independently

selected from the group consisting of $C_6$-$C_{36}$ aryl and $C_3$-$C_{36}$ heteroaryl; and

FL is substituted at the 9-position of the fluorenyl with two hydrocarbyl groups which may be linked with each other to form a ring.

According to one embodiment, there is provided a compound of the following formula (I)

A-$(Cy)_n$-Pz-FL (I)

wherein

A is selected from naphtalenyl, anthracenyl, phenanthrenyl, triphenylenyl, acenaphtenyl, fluoranthenyl, pyrenyl, tetracenyl, pyridinyl, pyrazinyl, pyrimidinyl, pyridazinyl, triazinyl, quinolinyl, isoquinolinyl, cinnolinyl, phtalazinyl, quinazolinyl, quinoxalinyl, naphtyridinyl, imidazopyridinyl, phenathridinyl, phenanthrolinyl, acridinyl, benzoacridinyl, dibenzoacridinyl, which are substituted or unsubstituted, respectively;
wherein, if A is substituted, the substituent(s) are independently selected from the group from the group consisting of $C_6$-$C_{18}$ aryl;

n is 1 or 2;

Cy is independently selected from the group consisting of $C_6$-$C_{12}$ aryl and $C_3$-$C_{12}$ heteroaryl;
wherein, if Cy is substituted, the substituent(s) are independently selected from the groups consisting of consisting of D and $C_1$-$C_4$ alkyl;

Pz is fully substituted pyrazinylene, wherein the substituents of the fully substituted pyrazinylene are independently selected from the group consisting of $C_6$-$C_{36}$ aryl; and

FL is 9,9'-$C_1$ to $C_{10}$-dialkyl-fluorenyl.

According to one embodiment, there is provided a compound of the following formula (I)

A-$(Cy)_n$-Pz-FL (I)

wherein

A is selected from pyridinyl, pyrazinyl, pyrimidinyl, pyridazinyl or triazinyl, especially from pyrimidinyl or triazinyl, which are substituted or unsubstituted, respectively;

wherein, if A is substituted, the substituent(s) are independently selected from the group from the group consisting of $C_6$-$C_{12}$ aryl, especially are phenyl, respectively;

n is 1;

Cy is independently selected from the group consisting of unsubstituted phenylene and unsubstituted naphthenylene, especially may be unsubstituted phenylene, such as meta-phenylene;

Pz is fully substituted pyrazinylene, wherein the substituents of the fully substituted pyrazinylene are independently selected from the group consisting of $C_6$-$C_{12}$ aryl, especially are phenyl, respectively; and

FL is 9,9'-$C_1$ to $C_4$-dialkyl-fluorenyl, especially 9,9'-dimethyl-fluorenyl.

The object is further achieved by an organic semiconducting material comprising the compound of formula (I) in accordance with the present invention.

The organic semiconducting material may, in addition to the compound of formula (I) comprise an electrical dopant, especially a redox n-dopant.

Under redox n-dopant, it is understood a compound which, if embedded into an electron transport matrix, improves, in comparison with the neat matrix under the same physical condictions, the electron properties of the formed organic material, particularly in terms of electron injection and/or electron conductivity.

In the context of the present invention "embedded into an electron transport matrix" means homogenously mixed with the electron transport matrix.

The redox n-dopant may be selected from elemental metals, metal salts, metal complexes and organic radicals.

In one embodiment, the redox n-dopant is selected from alkali metal salts and alkali metal complexes; preferably from lithium salts and lithium organic complexes; more preferably from lithium halides and lithium organic chelates; even more preferably from lithium fluoride, a lithium quinolinolate, lithium borate, lithium phenolate, lithium pyridinolate or from a lithium complex with a Schiff base ligand; most preferably,

- the lithium complex has the formula II, III or IV:

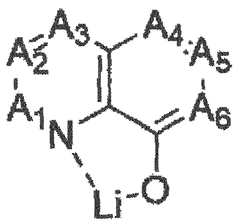 (II), 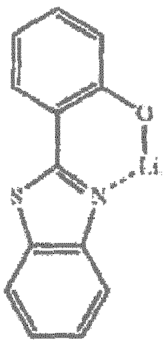 (III), 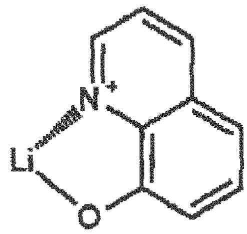 (IV)

wherein

$A_1$ to $A_6$ are same or independently selected from CH, CR, N, O;

R is same or independently selected from hydrogen, halogen, alkyl or aryl or heteroaryl with 1 to 20 carbon atoms; and more preferred $A_1$ to $A_6$ are CH,

- the borate based organic ligand is a tetra(1H-pyrazol-1-yl)borate,
- the phenolate is a 2-(pyridin-2-yl)phenolate, a 2-(diphenylphosphoryl)phenolate, an imidazol phenolate, 2-(pyridin-2-yl)phenolate or 2-(1-phenyl-1H-benzo[d]imidazol-2-yl)phenolate,
- the pyridinolate is a 2-(diphenylphosphoryl)pyridin-3-olate,
- the lithium Schiff base has the structure 100, 101, 102 or 103:

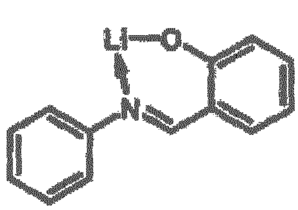 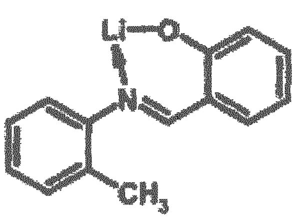 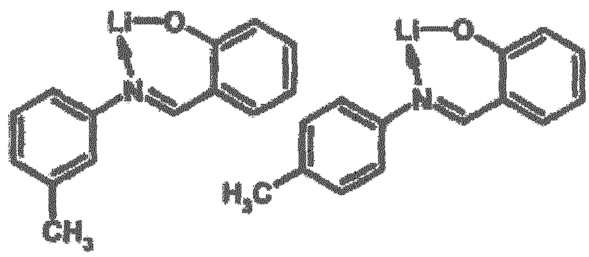

100 101 102 103

According to one embodiment of the invention, the organic semiconducting material of the present invention comprises a lithium organic complex, alternatively LiQ.

According to one embodiment of the present invention the organic semiconducting material comprises a metal, preferably selected from alkali metals, alkaline earth metals, rare earth metals and metals of the first transition period Ti, V, Cr and Mn, especially selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The most practical benchmark for the strength of an n-dopant is the value of its redox potential. There is no particular limitation in terms how negative the value of the redox potential can be.

As reduction potentials of usual electron transport matrices used in organic semiconductors are, if measured by cyclic voltammetry against ferrocene/ferrocenium reference redox couple, roughly in the range from about - 0.8 V to about - 3.1V; the practically applicable range of redox potentials for n-dopants which can effectively n-dope such matrices is in a slightly broader range, from about - 0.5 to about - 3.3 V.

The measurement of redox potentials is practically performed for a corresponding redox couple consisting of the reduced and of the oxidized form of the same compound.

In case that the redox n-dopant is an electrically neutral metal complex and/or an electrically neutral organic radical, the measurement of its redox potential is actually performed for the redox couple formed by

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

Preferably, the redox potential of the electrically neutral metal complex and/or of the electrically neutral organic radical may have a value which is more negative than - 0.5 V, preferably more negative than -1.2 V, more preferably more negative than - 1.7 V, even more preferably more negative than - 2.1 V, most preferably more negative than - 2.5 V, if measured by cyclic voltammetry against ferrocene/ferrocenium reference redox couple for a corresponding redox couple consisting of

(i) the electrically neutral metal complex and its cation radical formed by an abstraction of one electron from the electrically neutral metal complex, or

(ii) the electrically neutral organic radical and its cation formed by an abstraction of one electron from the electrically neutral organic radical.

In a preferred embodiment, the redox potential of the n-dopant is between the value which is about 0.5 V more positive and the value which is about 0.5 V more negative than the value of the reduction potential of the chosen electron transport matrix.

Electrically neutral metal complexes suitable as redox n-dopants may be e.g. strongly reductive complexes of some transition metals in low oxidation state. Particularly strong redox n-dopants may be selected for example from Cr(II), Mo(II) and/or W(II) guanidinate complexes such as W2(hpp)4, as described in more detail in WO2005/086251.

Electrically neutral organic radicals suitable as redox n-dopants may be e.g. organic radicals created by supply of additional energy from their stable dimers, oligomers or polymers, as described in more detail in EP 1 837 926 B1, WO2007/107306, or WO2007/107356. Under an elemental metal, it is understood a metal in a state of a neat metal, of a metal alloy, or in a state of free atoms or metal clusters. It is understood that metals deposited by vacuum thermal evaporation from a metallic phase, e.g. from a neat bulk metal, vaporize in their elemental form. It is further understood that if the vaporized elemental metal is deposited together with a covalent matrix, the metal atoms and/or clusters are embedded in the covalent matrix. In other words, it is understood that any metal doped covalent material prepared by vacuum thermal evaporation contains the metal at least partially in its elemental form.

For the use in consumer electronics, only metals containing stable nuclides or nuclides having very long halftime of radioactive decay might be applicable. As an acceptable level of nuclear stability, the nuclear stability of natural potassium can be taken.

In one embodiment, the n-dopant may be selected from electropositive metals selected from alkali metals, alkaline earth metals, rare earth metals and metals of the first transition period Ti, V, Cr and Mn. Preferably, the n-dopant may be selected from Li, Na, K, Rb, Cs, Mg, Ca, Sr, Ba, Sm, Eu, Tm, Yb; more preferably from Li, Na, K, Rb, Cs, Mg and Yb, even more preferably from Li, Na, Cs and Yb, most preferably from Li, Na and Yb.

The object is further achieved by an organic electronic device comprising a semiconductor layer, wherein the semiconductor layer comprises the compound of formula (I) in accordance with the present invention, especially comprises or consists of the organic semiconducting material according to the invention.

According to one embodiment of the invention, the organic semiconductor layer of the present invention is an electron transport layer, an electron injection layer or charge generation layer; alternatively an electron transport layer or a charge generation layer.

The organic electronic device according to the invention may be an organic light emitting diode. The present invention furthermore relates to a display device comprising an organic electronic device according to the present invention.

Finally, the object is achieved by a process for preparing an organic electronic device according to the invention

comprising a step of depositing the compound according to the invention on a solid support. In this regard, the "solid support" may be any solid surface, especially may be a layer of the organic electronic device which is arranged adjacent to the organic semiconductor layer in the organic electronic device.

Further layers

In accordance with the invention, the organic electronic device may comprise, besides the layers already mentioned above, further layers. Exemplary embodiments of respective layers are described in the following:

*Substrate*

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate or a silicon substrate.

*Anode electrode*

Either a first electrode or a second electrode comprised in the inventive organic electronic device may be an anode electrode. The anode electrode may be formed by depositing or sputtering a material that is used to form the anode electrode. The material used to form the anode electrode may be a high work-function material, so as to facilitate hole injection. The anode material may also be selected from a low work function material (i.e. aluminum). The anode electrode may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode electrode. The anode electrode may also be formed using metals, typically silver (Ag), gold (Au), or metal alloys.

*Hole injection layer*

A hole injection layer (HIL) may be formed on the anode electrode by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10-8 to 10-3 Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto. The HIL may be selected from a hole-transporting matrix compound doped with a p-type dopant. Typical examples of known doped hole transport materials are: copper phthalocyanine (CuPc), which HOMO level is approximately -5.2 eV, doped with tetrafluoro-tetracyanoquinonedimethane (F4TCNQ), which LUMO level is about -5.2 eV; zinc phthalocyanine (ZnPc) (HOMO = -5.2 eV) doped with F4TCNQ; a-NPD (N,N'-Bis(naphthalen-1-yl)-NN-bis(phenyl)-benzidine) doped with F4TCNQ. $\alpha$-NPD doped with 2,2'-(perfluoronaphthalen-2,6-diylidene) dimalononitrile. The p-type dopant concentrations can be selected from 1 to 20 wt.-%, more preferably from 3 wt.-% to 10 wt.-%.

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in driving voltage.

*Hole transport layer*

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

The HTL may be formed of any compound that is commonly used to form a HTL. Compounds that can be suitably used are disclosed for example in Yasuhiko Shirota and Hiroshi Kageyama, Chem. Rev. 2007, 107, 953-1010 and incorporated by reference. Examples of the compound that may be used to form the HTL are: carbazole derivatives, such as N-phenylcarbazole or polyvinylcarbazole; benzidine derivatives, such as N,N'-bis(3-methylphenyl)-N,N'-diphenyl-[1,1-biphenyl]-4,4'-diamine (TPD), or N,N'-di(naphthalen-i-yl)-N,N'-diphenyl benzidine (alpha-NPD); and triphenylamine-based compound, such as 4,4',4"-tris(N-carbazolyl)triphenylamine (TCTA). Among these compounds, TCTA can transport holes and inhibit excitons from being diffused into the EML.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in driving voltage.

*Electron blocking layer*

The function of an electron blocking layer (EBL) is to prevent electrons from being transferred from an emission layer to the hole transport layer and thereby confine electrons to the emission layer. Thereby, efficiency, operating voltage and/or lifetime are improved. Typically, the electron blocking layer comprises a triarylamine compound. The triarylamine compound may have a LUMO level closer to vacuum level than the LUMO level of the hole transport layer. The electron blocking layer may have a HOMO level that is further away from vacuum level compared to the HOMO level of the hole transport layer. The thickness of the electron blocking layer may be selected between 2 and 20 nm.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer is selected from triarylamine compounds with a triplet level above the triplet level of the phosphorescent emitter in the adjacent emission layer. Suitable compounds for the triplet control layer, in particular the triarylamine compounds, are described in EP 2 722 908 A1.

*Photoactive layer (PAL)*

The photoactive layer converts an electrical current into photons or photons into an electrical current.

The PAL may be formed on the HTL by vacuum deposition, spin coating, slot-die coat-ing, printing, casting, LB deposition, or the like. When the PAL is formed using vacuum deposition or spin coating, the conditions for deposition and coating may be similar to those for the formation of the HIL. However, the conditions for deposition and coating may vary, according to the compound that is used to form the PAL.

It may be provided that the photoactive layer does not comprise the compound of Formula (I).

The photoactive layer may be a light-emitting layer or a light-absorbing layer.

*Emission layer (EML)*

The EML may be formed on the HTL by vacuum deposition, spin coating, slot-die coat-ing, printing, casting, LB deposition, or the like. When the EML is formed using vacuum deposition or spin coating, the conditions for deposition and coating may be similar to those for the formation of the HIL. However, the conditions for deposition and coating may vary, according to the compound that is used to form the EML.

It may be provided that the emission layer does not comprise the compound of Formula (I).

The emission layer (EML) may be formed of a combination of a host and an emitter dopant. Example of the host are Alq3, 4,4'-N,N'-dicarbazole-biphenyl (CBP), poly(n-vinylcarbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters

which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp2lr(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F2Irpic, (F2ppy)2Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in driving voltage.

Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the ETL. When the EML comprises a phosphorescent dopant, the HBL may have also a triplet exciton blocking function. The hole blocking layer may be the inventive organic semiconductor layer comprising or consisting of the inventive compound represented by the general Formula (I) as defined above.

The HBL may also be named auxiliary ETL or a-ETL.

When the HBL is formed using vacuum deposition or spin coating, the conditions for deposition and coating may be similar to those for the formation of the HIL. However, the conditions for deposition and coating may vary, according to the compound that is used to form the HBL. Any compound that is commonly used to form a HBL may be used. Examples of compounds for forming the HBL include oxadiazole derivatives, triazole derivatives, and phenanthroline derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

The hole blocking layer may also be described as a-ETL or auxiliary ETL.

According to an embodiment, a hole blocking layer is arranged between the at least one photoactive layer and the organic semiconductor layer comprising compound of Formula (I).

According to an embodiment, a hole blocking layer is arranged between the at least one photoactive layer and the organic semiconductor layer comprising compound of Formula (I), wherein the organic semiconductor layer comprising compound of Formula (I) further comprises a redox n-dopant.

According to an embodiment, a hole blocking layer is arranged between the at least one photoactive layer and the organic semiconductor layer comprising compound of Formula (I), wherein the organic semiconductor layer comprising compound of Formula (I) further comprises a metal or a metal organic complex, alternatively a metal or a lithium organic complex.

According to an embodiment, a hole blocking layer is arranged between the at least one photoactive layer and the organic semiconductor layer comprising compound of Formula (I), wherein the organic semiconductor layer comprising compound of Formula (I) further comprises a metal.

*Electron transport layer (ETL)*

The OLED according to the present invention may comprise an electron transport layer (ETL). In accordance with one preferred embodiment of the invention, the electron transport layer may be the inventive organic semiconductor layer comprising the inventive compound represented by the general Formula (I) as defined herein.

According to various embodiments the OLED may comprise an electron transport layer or an electron transport layer stack comprising at least a first electron transport layer and at least a second electron transport layer.

By suitably adjusting energy levels of particular layers of the ETL, the injection and transport of the electrons may be controlled, and the holes may be efficiently blocked. Thus, the OLED may have long lifetime.

The electron transport layer of the organic electronic device may comprise the compound represented by general Formula (I)as defined above as the organic electron transport matrix (ETM) material. The electron transport layer may comprise, besides or instead of the compound represented by the general Formula (I), further ETM materials known in the art. Likewise, the electron transport layer may comprise as the only electron transport matrix material the compound represented by general Formula (I). In case that the inventive organic electronic device comprises more than

one electron transport layers, the compound represented by the general Formula (I) may be comprised in only one of the electron transport layers, in more than one of the electron transport layers or in all of the electron transport layers. In accordance with the invention, the electron transport layer may comprise, besides the ETM material, at least one additive as defined below.

Further, the electron transport layer may comprise one or more n-type dopants. The additive may be an n-type dopant. The additive can be alkali metal, alkali metal compound, alkaline earth metal, alkaline earth metal compound, transition metal, transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. In another emdodiment, the n-type dopant can be one selected from a group consisting of Cs, K, Rb, Mg, Na, Ca, Sr, Eu and Yb. In an embodiment the alkali metal compound may be 8-Hydroxyquinolinolato-lithium (LiQ), Lithium tetra(1H-pyrazol-1-yl)borate or Lithium 2-(diphenylphosphoryl)phenolate. Suitable compounds for the ETM (which may be used in addition to the inventive compound represented by the general Formula (I)as defined above) are not particularly limited. In one embodiment, the electron transport matrix compounds consist of covalently bound atoms. Preferably, the electron transport matrix compound comprises a conjugated system of at least 6, more preferably of at least 10 delocalized electrons. In one embodiment, the conjugated system of delocalized electrons may be comprised in aromatic or heteroaromatic structural moieties, as disclosed e.g. in documents EP 1970 371 A1 or WO 2013/079217 A1.

*Electron injection layer (EIL)*

An optional EIL, which may facilitates injection of electrons from the cathode, may be formed on the ETL, preferably directly on the electron transport layer. Examples of materials for forming the EIL include lithium 8-hydroxyquinolinolate (LiQ), LiF, NaCl, CsF, Li2O, BaO, Ca, Ba, Yb, Mg which are known in the art. Deposition and coating conditions for forming the EIL are similar to those for formation of the HIL, although the deposition and coating conditions may vary, according to the material that is used to form the EIL. The EIL may be the organic semiconductor layer comprising the compound of Formula (I).

The thickness of the EIL may be in the range from about 0.1 nm to about 10 nm, for example, in the range from about 0.5 nm to about 9 nm. When the thickness of the EIL is within this range, the EIL may have satisfactory electron-injecting properties, without a substantial penalty in driving voltage.

*Cathode electrode*

The cathode electrode is formed on the EIL if present. The cathode electrode may be formed of a metal, an alloy, an electrically conductive compound, or a mixture thereof. The cathode electrode may have a low work function. For example, the cathode electrode may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode electrode may be formed of a transparent conductive oxide, such as ITO or IZO.

The thickness of the cathode electrode may be in the range from about 5 nm to about 1000 nm, for example, in the range from about 10 nm to about 100 nm. When the thickness of the cathode electrode is in the range from about 5 nm to about 50 nm, the cathode electrode may be transparent or semitransparent even if formed from a metal or metal alloy.

It is to be understood that the cathode electrode is not part of an electron injection layer or the electron transport layer.

*Charge generation layer (CGL)*

The charge generation layer (CGL) may comprise a p- type charge generation layer (p-CGL) and an n-type charge generation layer (n-CGL). An interlayer may be arranged between the p-CGL and the -n-CGL.

Typically, the charge generation layer is a pn junction joining an n-type charge generation layer (electron generating layer) and a hole generating layer. The n-side of the pn junction generates electrons and injects them into the layer which is adjacent in the direction to the anode. Analogously, the p-side of the p-n junction generates holes and injects them into the layer which is adjacent in the direction to the cathode.

Charge generating layers are used in tandem and stacked devices, for example, in tandem or stacked OLEDs comprising, between two electrodes, two or more emission layers. In a tandem or stacked OLED comprising two emission layers, the n-type charge generation layer provides electrons for the first light emission layer arranged near the anode, while the hole generating layer provides holes to the second light emission layer arranged between the first emission layer and the cathode.

Suitable matrix materials for the hole generating layer may be materials conventionally used as hole injection and/or hole transport matrix materials. Also, p-type dopant used for the hole generating layer can employ conventional

materials. For example, the p-type dopant can be one selected from a group consisting of tetrafluore-7,7,8,8-tetracyanoquinodimethane (F4-TCNQ), derivatives of tetracyanoquinodimethane, radialene derivatives, iodine, FeCl3, FeF3, and SbCl5. Also, the host can be one selected from a group consisting of N,N'-di(naphthalen-1-yl)-N,N-diphenyl-benzidine (NPB), N,N'-diphenyl-N(N'-bis(3-methylphenyl)-1,1-biphenyl-4,4'-diamine (TPD) and N,N',N'-tetranaphthyl-benzidine (TNB). The p-type charge generation layer may consist of CNHAT.

The n-type charge generating layer may be the layer comprising the compound of Formula (I). The n-type charge generation layer can be layer of a neat n-type dopant, for example of a metal, or can consist of an organic matrix material doped with the n-type dopant. In one embodiment, the n-type dopant can be alkali metal, alkali metal compound, alkaline earth metal, alkaline earth metal compound, a transition metal, a transition metal compound or a rare earth metal. In another embodiment, the metal can be one selected from a group consisting of Li, Na, K, Rb, Cs, Mg, Ca, Sr, Ba, La, Ce, Sm, Eu, Tb, Dy, and Yb. More specifically, the n-type dopant can be one selected from a group consisting of Li, Cs, K, Rb, Mg, Na, Ca, Sr, Eu and Yb. Suitable matrix materials for the electron generating layer may be the materials conventionally used as matrix materials for electron injection or electron transport layers. The matrix material can be for example one selected from a group consisting of triazine compounds, hydroxyquinoline derivatives like tris(8-hydroxyquinoline)aluminum, benzazole derivatives, and silole derivatives.

The hole generating layer is arranged in direct contact to the n-type charge generation layer.

According to one aspect of the present invention, the organic semiconductor layer is arranged between the first and second emission layer and further comprises a redox n-dopant.

According to one aspect of the present invention, the organic semiconductor layer is arranged between the first and second emission layer and further comprises a metal.

According to one aspect of the present invention, the organic semiconductor layer is arranged between the first and second emission layer and further comprises a metal selected from alkali, alkaline earth and rare earth metals.

According to one aspect of the present invention, the organic semiconductor layer comprising compound of Formula (I) is arranged between the first and second emission layer and a further organic semiconductor layer comprising compound of Formula (I) is arranged between the second emission layer and the cathode.

According to one aspect of the present invention, the organic semiconductor layer comprising compound of Formula (I) is arranged between the first and second emission layer and a further organic semiconductor layer comprising compound of Formula (I) is arranged between the second emission layer and the cathode; wherein the organic semiconductor layer comprising compound of Formula (I) further comprises a redox n-dopant.

According to one aspect of the present invention, the organic semiconductor layer comprising compound of Formula (I) is arranged between the first and second emission layer and a further organic semiconductor layer comprising compound of Formula (I) is arranged between the second emission layer and the cathode; wherein the further organic semiconductor layer comprising compound of Formula (I) further comprises a redox n-dopant.

According to one aspect of the present invention, the organic semiconductor layer comprising compound of Formula (I) is arranged between the first and second emission layer and a further organic semiconductor layer comprising compound of Formula (I) is arranged between the second emission layer and the cathode; wherein the organic semiconductor layer comprising compound of Formula (I) further comprises a redox n-dopant, and the further organic semiconductor layer comprising compound of Formula (I) further comprises a redox n-dopant.

According to one aspect of the present invention, the organic semiconductor layer comprising compound of Formula (I) is arranged between the first and second emission layer and a further organic semiconductor layer comprising compound of Formula (I) is arranged between the second emission layer and the cathode; wherein the organic semiconductor layer comprising compound of Formula (I) further comprises a metal, and the further organic semiconductor layer comprising compound of Formula (I) further comprises a redox n-dopant.

## Organic light-emitting diode

The organic electronic device according to the invention may be an organic light-emitting device.

According to one aspect of the present invention, there is provided an organic light-emitting diode (OLED) comprising: a substrate; an anode electrode formed on the substrate; a hole injection layer, a hole transport layer, an emission layer, an organic semiconductor layer comprising a compound of Formula (I)and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (I) and a cathode electrode.

According to another aspect of the present invention, there is provided an OLED comprising: a substrate; an anode electrode formed on the substrate; a hole injection layer, a hole transport layer, an electron blocking layer, an emission layer, a hole blocking layer, an organic semiconductor layer comprising a compound of Formula (I), an electron injection layer, and a cathode electrode.

According to various embodiments of the present invention, there may be provided OLEDs layers arranged between the above mentioned layers, on the substrate or on the top electrode.

According to one aspect, the OLED can comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a hole generating layer, the hole generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional injection layer are arranged.

The organic semiconductor layer according to the invention may be the electron transport layer, first electron transport layer, n-type charge generation layer and/or second electron transport layer.

For example, the OLED according to Fig. 2 may be formed by a process, wherein on a substrate (110), an anode (120), a hole injection layer (130), a hole transport layer (140), an electron blocking layer (145), an emission layer (150), a hole blocking layer (155), an electron transport layer (160), an electron injection layer (180) and the cathode electrode (190) are subsequently formed in that order.

Organic electronic device

The organic electronic device according to the invention may be a light emitting device, or a photovoltaic cell, and preferably a light emitting device.

According to another aspect of the present invention, there is provided a method of manufacturing an organic electronic device, the method using:

- at least one deposition source, preferably two deposition sources and more preferred at least three deposition sources.

The methods for deposition that can be suitable comprise:

- deposition via vacuum thermal evaporation;
- deposition via solution processing, preferably the processing is selected from spin-coating, printing, casting; and/or
- slot-die coating.

According to various embodiments of the present invention, there is provided a method using:

- a first deposition source to release the compound of Formula (I) according to the invention, and
- a second deposition source to release the metal, a metal salt or an alkali or alkaline earth metal complex; alternatively an organic alkali or alkaline earth metal complex; alternatively 8-hydroxyquinolinolato lithium;

the method comprising the steps of forming the organic semiconductor layer; whereby for an organic light-emitting diode (OLED):

- the organic semiconductor layer is formed by releasing the compound of Formula (I) according to the invention from the first deposition source and a metal, a metal salt or an alkali or alkaline earth metal complex; alternatively an organic alkali or alkaline earth metal complex; alternatively 8-hydroxyquinolinolato lithium, from the second deposition source.

According to various embodiments of the present invention, the method may further include forming on the anode electrode, an emission layer and at least one layer selected from the group consisting of forming a hole injection layer, forming a hole transport layer, or forming a hole blocking layer, between the anode electrode and the first electron transport layer.

According to various embodiments of the present invention, the method may further include the steps for forming an organic light-emitting diode (OLED), wherein

- on a substrate a first anode electrode is formed,
- on the first anode electrode an emission layer is formed,
- on the emission layer an electron transport layer stack is formed, optionally a hole blocking layer is formed on the emission layer and an organic semiconductor layer is formed,
- and finally a cathode electrode is formed,
- optional a hole injection layer, a hole transport layer, and a hole blocking layer, formed in that order between the first anode electrode and the emission layer,
- optional an electron injection layer is formed between the organic semiconductor layer and the cathode electrode.

According to various embodiments of the present invention, the method may further comprise forming an electron injection layer on the organic semiconductor layer. However, according to various embodiments of the OLED of the present invention, the OLED may not comprise an electron injection layer.

According to various embodiments, the OLED may have the following layer structure, wherein the layers having the following order:
anode, hole injection layer, first hole transport layer, second hole transport layer, emission layer, optional hole blocking layer, organic semiconductor layer comprising a compound of Formula (I) according to the invention, optional electron injection layer, and cathode.

According to another aspect of the invention, it is provided an electronic device comprising at least one organic light emitting device according to any embodiment described throughout this application, preferably, the electronic device comprises the organic light emitting diode in one of embodiments described throughout this application. More preferably, the electronic device is a display device.

In one embodiment, the organic electronic device according to the invention comprising an organic semiconductor layer comprising a compound according to Formula (I) may further comprise a layer comprising a radialene compound and/or a quinodimethane compound.

In one embodiment, the radialene compound and/or the quinodimethane compound may be substituted with one or more halogen atoms and/or with one or more electron withdrawing groups. Electron withdrawing groups can be selected from nitrile groups, halogenated alkyl groups, alternatively from perhalogenated alkyl groups, alternatively from perfluorinated alkyl groups. Other examples of electron withdrawing groups may be acyl, sulfonyl groups or phosphoryl groups.

Alternatively, acyl groups, sulfonyl groups and/or phosphoryl groups may comprise halogenated and/or perhalogenated hydrocarbyl. In one embodiment, the perhalogenated hydrocarbyl may be a perfluorinated hydrocarbyl. Examples of a perfluorinated hydrocarbyl can be perfluormethyl, perfluorethyl, perfluorpropyl, perfluorisopropyl, perfluorobutyl, perfluorophenyl, perfluorotolyl; examples of sulfonyl groups comprising a halogenated hydrocarbyl may be trifluoromethylsulfonyl, pentafluoroethylsulfonyl, pentafluorophenylsulfonyl, heptafluoropropylsufonyl, nonafluorobutylsulfonyl, and like.

In one embodiment, the radialene and/or the quinodimethane compound may be comprised in a hole injection, hole transporting and/or a hole generation layer.

In one embodiment, the radialene compound may have Formula (XX) and/or the quinodimethane compound may have Formula (XXIa) or (XXIb):

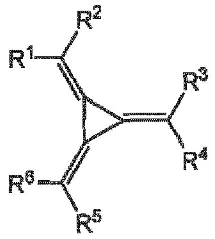
(XX)
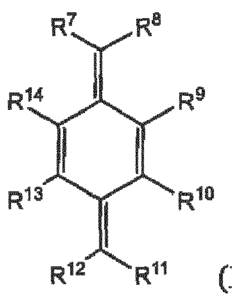
(XXIa)
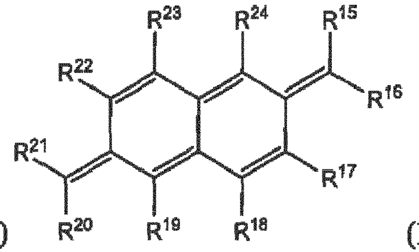
(XXIb),

wherein (as an exception different to the description above) $R^1$, $R^2$, $R^3$, $R^4$, $R^5$, $R^6$, $R^7$, $R^8$, $R^{11}$, $R^{12}$, $R^{15}$, $R^{16}$, $R^{20}$, $R^{21}$ are independently selected from above mentioned electron withdrawing groups and $R^9$, $R^{10}$, $R^{13}$, $R^{14}$, $R^{17}$, $R^{18}$, $R^{19}$,

$R^{22}$, $R^{23}$ and $R^{24}$ are independently selected from H, halogen and above mentioned electron withdrawing groups.

According to one embodiment of the present invention, the organic semiconductor layer comprising compound of Formula (I) is adjacent to a layer comprising a compound of formula (XX), (XXIa) or (XXIb).

According to one embodiment of the present invention, the organic semiconductor layer comprising compound of Formula (I) is in direct contact to a layer comprising a compound of formula (XX), (XXIa) or (XXIb).

GENERAL DEFINITIONS

In the present specification, when a definition is not otherwise provided, an "alkyl group" may refer to an aliphatic hydrocarbon group. The alkyl group may refer to "a saturated alkyl group" without any double bond or triple bond. The term "alkyl" as used herein shall encompass linear as well as branched and cyclic alkyl. For example, $C_3$-alkyl may be selected from n-propyl and iso-propyl. Likewise, $C_4$-alkyl encompasses n-butyl, sec-butyl and t-butyl. Likewise, $C_6$-alkyl encompasses n-hexyl and cyclo-hexyl.

As used herein if not explicitly mentioned else, the asterisk symbol "*" represents a binding position at which the moiety labelled accordingly is bond to another moiety.

The subscribed number n in $C_n$ relates to the total number of carbon atoms in the respective alkyl, arylene, heteroarylene or aryl group.

The term "aryl" or "arylene" as used herein shall encompass phenyl ($C_6$-aryl), fused aromatics, such as naphthalene, anthracene, phenanthrene, tetracene etc.. Further encompassed are biphenyl and oligo- or polyphenyls, such as terphenyl, phenyl-substituted biphenyl, phenyl-substituted terphenyl (such as tetraphenyl benzole groups) etc.. "Arylene" respectively "heteroarylene", refers to groups to which two further moieties are attached. In the present specification, the term "aryl group" or "arylene group" may refer to a group comprising at least one hydrocarbon aromatic moiety, and all the elements of the hydrocarbon aromatic moiety may have p-orbitals which form conjugation, for example a phenyl group, a napthyl group, an anthracenyl group, a phenanthrenyl group, a pyrinyl group, a fluorenyl group and the like. Further encompoassed are spiro compounds in which two aromatic moieties are connected with each other via a spiro-atom, such as 9,9'-spirobi[9H-fluorene]yl. The aryl or arylene group may include a monocyclic or fused ring polycyclic (i.e., links sharing adjacent pairs of carbon atoms) functional group.

The term "heteroaryl" as used herein refers to aryl groups in which at least one carbon atom is substituted with a heteroatom. The term "heteroaryl" may refer to aromatic heterocycles with at least one heteroatom, and all the elements of the hydrocarbon heteroaromatic moiety may have p-orbitals which form conjugation. The heteroatom may be selected from N, O, S, B, Si, P, Se, preferably from N, O and S. A heteroarylene ring may comprise at least 1 to 3 heteroatoms. Preferably, a heteroarylene ring may comprise at least 1 to 3 heteroatoms individually selected from N, S and/or O. Just as in case of "aryl"/"arylene", the term "heteroaryl" comprises, for example, spiro compounds in which two aromatic moieties are connected with each other, such as spiro[fluorene-9,9'-xanthene]. Further exemplary heteroaryl groups are diazine, triazine, dibenzofurane, dibenzothiofurane, acridine, benzoacridine, dibenzoacridine etc.

The term "alkenyl" as used herein refers to a group $-CR^1= CR^2R^3$ comprising a carbon-carbon double bond.

The term "perhalogenated" as used herein refers to a hydrocarbyl group wherein all of the hydrogen atoms of the hydrocarbyl group are replaced by halogen (F, Cl, Br, I) atoms.

The term "alkoxy" as used herein refers to a structural fragment of the Formula -OR with R being hydrocarbyl, preferably alkyl or cycloalkyl.

The term "thioalkyl" as used herein refers to a structural fragment of the Formula -SR with R being hydrocarbyl, preferably alkyl or cycloalkyl.

The subscripted number n in $C_n$-heteroaryl merely refers to the number of carbon atoms excluding the number of heteroatoms. In this context, it is clear that a $C_3$ heteroarylene group is an aromatic compound comprising three carbon atoms, such as pyrazol, imidazole, oxazole, thiazole and the like.

The term "heteroaryl" as used herewith shall encompass pyridine, quinoline, benzoquinoline, quinazoline, benzoquinazoline, pyrimidine, pyrazine, triazine, benzimidazole, benzothiazole, benzo[4,5]thieno[3,2-d]pyrimidine, carbazole, xanthene, phenoxazine, benzoacridine, dibenzoacridine and the like.

In the present specification, the term single bond refers to a direct bond.

The term "fluorinated" as used herein refers to a hydrocarbon group in which at least one of the hydrogen atoms comprised in the hydrocarbon group is substituted by a fluorine atom. Fluorinated groups in which all of the hydrogen atoms thereof are substituted by fluorine atoms are referred to as perfluorinated groups and are particularly addressed by the term "fluorinated".

In terms of the invention, a group is "substituted with" another group if one of the hydrogen atoms comprised in this group is replaced by another group, wherein the other group is the substituent.

In terms of the invention, the expression "between" with respect to one layer being between two other layers does not exclude the presence of further layers which may be arranged between the one layer and one of the two other layers. In terms of the invention, the expression "in direct contact" with respect to two layers being in direct contact with

each other means that no further layer is arranged between those two layers. One layer deposited on the top of another layer is deemed to be in direct contact with this layer.

The term "contacting sandwiched" refers to an arrangement of three layers whereby the layer in the middle is in direct contact with the two adjacent layers.

With respect to the inventive electron transport layer stack the compounds mentioned in the experimental part are most preferred.

A lighting device may be any of the devices used for illumination, irradiation, signaling, or projection. They are correspondingly classified as illuminating, irradiating, signaling, and projecting devices. A lighting device usually consists of a source of optical radiation, a device that transmits the radiant flux into space in the desired direction, and a housing that joins the parts into a single device and protects the radiation source and light-transmitting system against damage and the effects of the surroundings.

According to another aspect, the organic electroluminescent device according to the present invention comprises two or three or more emission layers. An OLED comprising more than one emission layer is also described as a tandem OLED or stacked OLED.

The organic electroluminescent device (OLED) may be a bottom- or top-emission device. The organic electroluminescent device (OLED) may emit the light trough a transparent anode or through a transparent cathode.

Another aspect is directed to a device comprising at least one organic electroluminescent device (OLED).

A device comprising organic light-emitting diodes is for example a display or a lighting panel.

In the present invention, the following defined terms, these definitions shall be applied, unless a different definition is given in the claims or elsewhere in this specification.

In the context of the present specification the term "different" or "differs" in connection with the matrix material means that the matrix material differs in their structural Formula.

The terms "OLED" and "organic light-emitting diode" are simultaneously used and have the same meaning. The term "organic electroluminescent device" as used herein may comprise both organic light emitting diodes as well as organic light emitting transistors (OLETs).

As used herein, "weight percent", "wt.-%", "percent by weight", "% by weight", and variations thereof refer to a composition, component, substance or agent as the weight of that component, substance or agent of the respective electron transport layer divided by the total weight of the respective electron transport layer thereof and multiplied by 100. It is under-stood that the total weight percent amount of all components, substances and agents of the respective electron transport layer and electron injection layer are selected such that it does not exceed 100 wt.-%.

As used herein, "volume percent", "vol.-%", "percent by volume", "% by volume", and variations thereof refer to a composition, component, substance or agent as the volume of that component, substance or agent of the respective electron transport layer divided by the total volume of the respective electron transport layer thereof and multiplied by 100. It is understood that the total volume percent amount of all components, substances and agents of the cathode layer are selected such that it does not exceed 100 vol.-%.

All numeric values are herein assumed to be modified by the term "about", whether or not explicitly indicated. As used herein, the term "about" refers to variation in the numerical quantity that can occur. Whether or not modified by the term "about" the claims include equivalents to the quantities.

It should be noted that, as used in this specification and the appended claims, the singular forms "a", "an", and "the" include plural referents unless the content clearly dictates otherwise.

The term "free of", "does not contain", "does not comprise" does not exclude impurities. Impurities have no technical effect with respect to the object achieved by the present invention.

In the context of the present specification the term "essentially non-emissive" or "non-emissive" means that the contribution of the compound or layer to the visible emission spectrum from the device is less than 10 %, preferably less than 5 % relative to the visible emission spectrum. The visible emission spectrum is an emission spectrum with a wavelength of about $\geq$ 380 nm to about $\leq$ 780 nm.

Preferably, the organic semiconducting layer comprising the compound of Formula (I) is essentially non-emissive or non-emitting.

The operating voltage, also named U, is measured in Volt (V) at 10 milliAmpere per square centimeter (mA/cm2).

The candela per Ampere efficiency, also named cd/A efficiency is measured in candela per ampere at 10 milliAmpere per square centimeter (mA/cm2).

The external quantum efficiency, also named EQE, is measured in percent (%).

The color space is described by coordinates CIE-x and CIE-y (International Commission on Illumination 1931). For blue emission the CIE-y is of particular importance. A smaller CIE-y denotes a deeper blue color. Efficiency values are compared at the same CIE-y.

The highest occupied molecular orbital, also named HOMO, and lowest unoccupied molecular orbital, also named LUMO, are measured in electron volt (eV).

The term "OLED", "organic light emitting diode", "organic light emitting device", "organic optoelectronic device"

and "organic light-emitting diode" are simultaneously used and have the same meaning.

The term "life-span" and "lifetime" are simultaneously used and have the same meaning.

The anode and cathode may be described as anode electrode / cathode electrode or anode electrode / cathode electrode or anode electrode layer / cathode electrode layer.

Room temperature, also named ambient temperature, is 23°C.

Hereinafter, the embodiments are illustrated in more detail with reference to examples. However, the present disclosure is not limited to the following examples. Reference will now be made in detail to the exemplary aspects.

DESCRIPTION OF THE DRAWINGS

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object of the invention are disclosed in the dependent claims and the following description of the respective figures which in an exemplary fashion show preferred embodiments according to the invention. Any embodiment does not necessarily represent the full scope of the invention, however, and reference is made therefore to the claims and herein for interpreting the scope of the invention. It is to be understood that both the foregoing general description and the following detailed description are exemplary and explanatory only and are intended to provide further explanation of the present invention as claimed.

FIG. 1 is a schematic sectional view of an organic electronic device, according to an exemplary embodiment of the present invention;

FIG. 2 is a schematic sectional view of an organic light-emitting diode (OLED), according to an exemplary embodiment of the present invention;

FIG. 3 is a schematic sectional view of an OLED, according to an exemplary embodiment of the present invention.

FIG. 4 is a schematic sectional view of an OLED comprising a charge generation layer and two emission layers, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

FIG. 1 is a schematic sectional view of an organic electronic device 100, according to an exemplary embodiment of the present invention. The organic electronic device 100 includes a substrate 110, an anode 120, a photoactive layer (PAL) 125, an organic semiconductor layer comprising a compound of formula (1) 160. The organic semiconductor layer comprising compound of formula (1) 160 is formed on the PAL 125. Onto the organic semiconductor layer 160, a cathode 190 is disposed.

FIG. 2 is a schematic sectional view of an organic light-emitting diode (OLED) 100, according to an exemplary embodiment of the present invention. The OLED 100 includes a substrate 110, an anode 120, a hole injection layer (HIL) 130, a hole transport layer (HTL) 140, an emission layer (EML) 150, an electron transport layer (ETL) 160. The electron transport layer (ETL) 160 is formed on the EML 150. Onto the electron transport layer (ETL) 160, an electron injection layer (EIL) 180 is disposed. The cathode 190 is disposed directly onto the electron injection layer (EIL) 180.

Instead of a single electron transport layer 160, optionally an electron transport layer stack (ETL) can be used.

Fig. 3 is a schematic sectional view of an OLED 100, according to another exemplary embodiment of the present invention. Fig. 3 differs from Fig. 2 in that the OLED 100 of Fig. 3 comprises an electron blocking layer (EBL) 145 and a hole blocking layer (HBL) 155.

Referring to Fig. 3, the OLED 100 includes a substrate 110, an anode 120, a hole injection layer (HIL) 130, a hole transport layer (HTL) 140, an electron blocking layer (EBL) 145, an emission layer (EML) 150, a hole blocking layer (HBL) 155, an electron transport layer (ETL) 160, an electron injection layer (EIL) 180 and a cathode electrode 190.

Preferably, the organic semiconductor layer comprising a compound of Formula (1) may be an ETL.

Fig. 4 is a schematic sectional view of an OLED 100, according to another exemplary embodiment of the present invention. Fig. 4 differs from Fig. 3 in that the OLED 100 of Fig. 4 further comprises a charge generation layer (CGL)

and a second emission layer (151).

Referring to Fig. 4, the OLED 100 includes a substrate 110, an anode 120, a first hole injection layer (HIL) 130, a first hole transport layer (HTL) 140, a first electron blocking layer (EBL) 145, a first emission layer (EML) 150, a first hole blocking layer (HBL) 155, a first electron transport layer (ETL) 160, an n-type charge generation layer (n-type CGL) 185, a hole generating layer (p-type charge generation layer; p-type GCL) 135, a second hole transport layer (HTL) 141, a second electron blocking layer (EBL) 146, a second emission layer (EML) 151, a second hole blocking layer (EBL) 156, a second electron transport layer (ETL) 161, a second electron injection layer (EIL) 181 and a cathode 190.

Preferably, the organic semiconductor layer comprising a compound of Formula (1) may be an n-type CGL.

Preferably, the organic semiconductor layer comprising a compound of Formula (1) may be the first ETL, n-type CGL and/or second ETL.

While not shown in Fig. 1, Fig. 2, Fig. 3 and Fig. 4, a sealing layer may further be formed on the cathode electrodes 190, in order to seal the OLEDs 100. In addition, various other modifications may be applied thereto.

Hereinafter, the embodiments are illustrated in more detail with reference to examples. However, the present disclosure is not limited to the following examples.

DETAILED DESCRIPTION

The invention is furthermore illustrated by the following examples which are illustrative only and non-binding.

EXPERIMENTAL PART

Melting point

The melting point (mp) is determined as peak temperatures from the DSC curves of the above TGA-DSC measurement or from separate DSC measurements (Mettler Toledo DSC822e, heating of samples from room temperature to completeness of melting with heating rate 10 K/min under a stream of pure nitrogen. Sample amounts of 4 to 6 mg are placed in a 40 μL Mettler Toledo aluminum pan with lid, a <1 mm hole is pierced into the lid).

Glass transition temperature

The glass transition temperature (Tg) is measured under nitrogen and using a heating rate of 10 K per min in a Mettler Toledo DSC 822e differential scanning calorimeter as described in DIN EN ISO 11357, published in March 2010.

Rate onset temperature

The rate onset temperature (TRO) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than $10^{-5}$ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 200 to 255 °C. If the rate onset temperature is below 200 °C the evaporation may be too rapid and therefore difficult to control. If the rate onset temperature is above 255 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

Reduction potential

The reduction potential is determined by cyclic voltammetry with potenioststic device Metrohm PGSTAT30 and software Metrohm Autolab GPES at room temperature. The redox potentials given at particular compounds were measured in an argon de-aerated, dry 0.1M THF solution of the tested substance, under argon atmosphere, with 0.1M tetrabutylammonium hexafluorophosphate supporting electrolyte, between platinum working electrodes and with an

Ag/AgCl pseudo-standard electrode (Metrohm Silver rod electrode), consisting of a silver wire covered by silver chloride and immersed directly in the measured solution, with the scan rate 100 mV/s. The first run was done in the broadest range of the potential set on the working electrodes, and the range was then adjusted within subsequent runs appropriately. The final three runs were done with the addition of ferrocene (in 0.1M concentration) as the standard. The average of potentials corresponding to cathodic and anodic peak of the studied compound, after subtraction of the average of cathodic and anodic potentials observed for the standard $Fc^+/Fc$ redox couple, afforded finally the values reported above. All studied compounds as well as the reported comparative compounds showed well-defined reversible electrochemical behavior.

Dipole moment

The dipole moment $|\vec{\mu}|$ of a molecule containing N atoms is given by:

$$\vec{\mu} = \sum_{i}^{N} q_i \vec{r_i}$$

$$|\vec{\mu}| = \sqrt{\mu_x^2 + \mu_y^2 + \mu_z^2}$$

where $q_i$ and $\vec{r_i}$ are the partial charge and position of atom i in the molecule.

The dipole moment is determined by a semi-empirical molecular orbital method.

The geometries of the molecular structures are optimized using the hybrid functional B3LYP with the 6-31G* basis set in the gas phase as implemented in the program package TURBOMOLE V6.5 (TURBOMOLE GmbH, Litzenhardtstrasse 19, 76135 Karlsruhe, Germany). If more than one conformation is viable, the conformation with the lowest total energy is selected to determine the bond lengths of the molecules.

Calculated HOMO and LUMP

The HOMO and LUMO are calculated with the program package TURBOMOLE V6.5 (TURBOMOLE GmbH, Litzenhardtstrasse 19, 76135 Karlsruhe, Germany). The optimized geometries and the HOMO and LUMO energy levels of the molecular structures are determined by applying the hybrid functional B3LYP with a 6-31G* basis set in the gas phase. If more than one conformation is viable, the conformation with the lowest total energy is selected.

Preparation of inventive compounds

**2-(3-(5-(9,9-dintethyl-9H-fluoren-2-yl)-3,6-diphenylpyrazin-2-yl)phenyl)-4,6-diphenylpyrimidine (E1)**

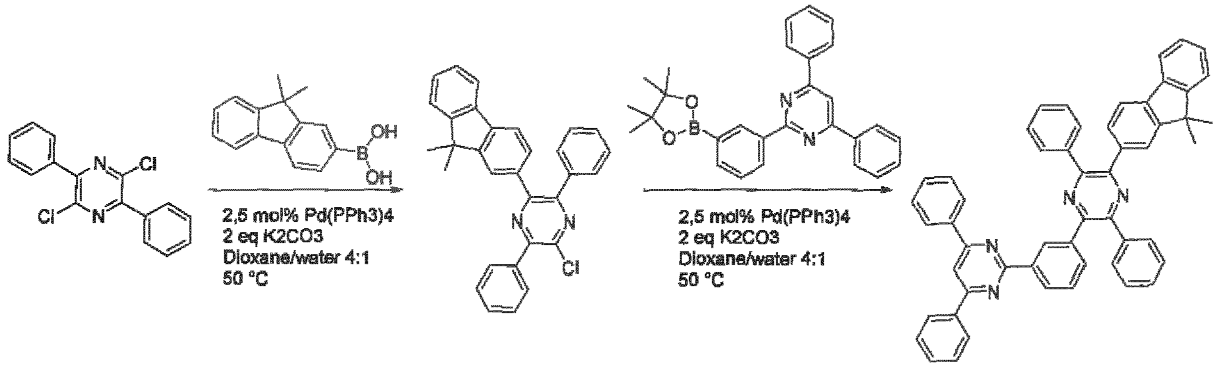

**1st Step. Synthesis of 2-chloro-5-(9,9-dimethyl-9H-fluoren-2-yl)-3,6-diphenylpyrazine**

A 3-neck round-bottom flask was flushed with nitrogen and charged with 32.80 g 2,5-dichloro-3,6-diphenylpyrazine (CAS 74134-61-5) (1 eq), 40.92 g (9,9-dimethyl-9H-fluoren-2-yl)boronic acid (CAS 333432-28-3) (1.6 eq), 3.15 g tetralds(triphenylphosphine)palladium(o) (CAS 14221-01-3) (0,025 eq), 30.11 g potassium carbonate (CAS 584-08-7) (2 eq). Deaerated mixture of 436 mL dioxane and 109 mL water was added. The reaction was running at 50 °C under nitrogen atmosphere overnight. Formed suspension was filtered off and solid was discarded. Filtrate was evaporated at low pressure, the raw product was dissolved in 400 mL chloroform and extracted with water. Combined organic phases were dried and filtered through silica pad. The solvent was evaporated and the crude product was washed with 300 mL acetonitrile at 80°C. Final purification by sublimation, yield: 19.84 g (39.2 %)

**2nd Step. Synthesis of 2-(3-(5-(9,9-dimethyl-9H-fluoren-2-yl)-3,6-diphenylpyrazin-2-yl)phenyl)-4,6-diphenylpyrimidine**

A 3-neck round-bottom flask was flushed with nitrogen and charged with 11.68 g 2-chloro-5-(9,9-dimethyl-9H-fluoren-2-yl)-3,6-diphenylpyrazine (no CAS) (1 eq), 10.49 g 4,6-diphenyl-2-(3-(4,4,5,5-tetramethyl-1,3,2-dioxaborolan-2-yl)phenyl)pyrimidine (CAS 1381862-91-4) (0.95 eq), 0.74 g tetrakis-(triphenylphosphine)palladium(o) (CAS 14221-01-3) (0.025 eq), 7.03 g potassium carbonate (CAS 584-08-7) (2 eq). Deaerated mixture of 100 mL dioxane and 25 mL water was added. The reaction was running at 50 °C under nitrogen atmosphere overnight. Next day, a yellow suspension formed. The precipitate was filtered and washed with dioxane, water, methanol and acetonitrile. The raw product was dissolved in 900 mL chloroform and filtered through silica pad. Final purification was done by sublimation. Yield: 12.33 g (66.24 %)

**2-(3-(3-(9,9-dimethyl-9H-fluoren-4-yl)-5,6-diphenylpyrazin-2-yl)phenyl)-4,6-diphenylpyrimidine (E2)**

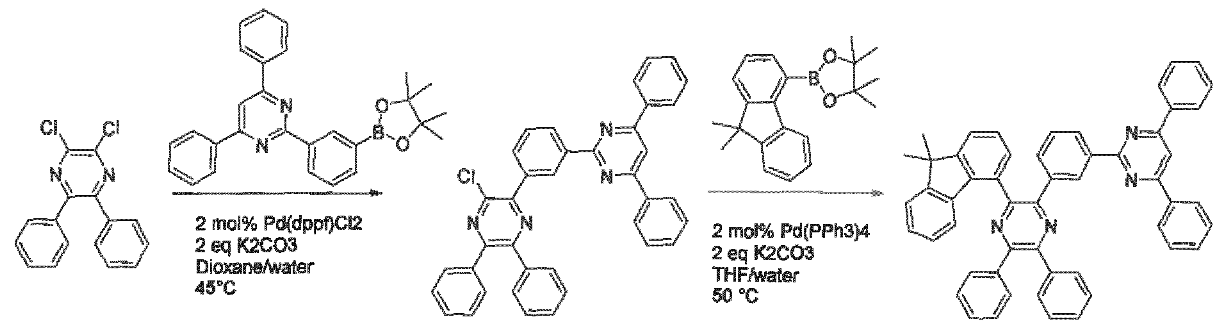


**1st Step. Synthesis of 2-(3-(3-chloro-5,6-diphenylpyrazin-2-yl)phenyl)-4,6-diphenylpyrimidine**

A 3-neck round-bottom flask was flushed with nitrogen and charged with 12.5 g 2,3-dichloro-5,6-diphenylpyrazine (CAS 57038-62-7) (1 eq), 19.83 g 4,6-diphenyl-2-(3-(4,4,5,5-tetramethyl-1,3,2-dioxaborolan-2-yl)phenyl)pyrimidine (CAS 1381862-91-4) (1.1 eq), 0.62 g [1,1'-bis(diphenylphosphino)ferrocene]-dichloropalladium(II) (CAS 72287-26-4) (0.02 eq), 11.46 g potassium carbonate (CAS 584-08-7) (2 eq). A deaerated mixture of 250 mL dioxane and 83 mL water was added. The reaction was running at 45 °C under a nitrogen atmosphere overnight. A yellow suspension formed. The solid precipitate was collected by filtration and washed with dioxane, water and methanol. Then, the solid was dissolved in 700 mL chloroform, the solution filtered through silica pad and evaporated. Final purification was done by sublimation. Yield: 11.11 g (48.43 %)

**2nd Step. Synthesis of 2-(3-(3-(9,9-dimethyl-9H-fluoren-4-yl)-5,6-diphenylpyrazin-2-yl)phenyl)-4,6-diphenylpyrimidine**

A 3-neck round-bottom flask was flushed with nitrogen and charged with 10.2 g 2-(3-(3-chloro-5,6-diphenylpyrazin-2-yl)phenyl)-4,6-diphenylpyrimidine (no CAS) (1 eq), 10.98 g 2-(9,9-dimethyl-9H-fluoren-4-yl)-4,4,5,5-tetramethyl-1,3,2-dioxaborolane (CAS 1365692-79-0) (1.93 eq), 0.513 g tetrakis(triphenyl-phosphine)palladium(o) (CAS 14221-01-3) (0.025 eq), 4.92 g potassium carbonate (CAS 584-08-7) (2 eq). A deaerated mixture of 72 mL THF and 18 mL water was added. The reaction was running at 50 °C under nitrogen atmosphere overnight. Next day, a dark suspension that formed was filtered and the solid was washed with THF, water and methanol. Then, the crude product was dissolved in 400 mL chloroform and filtered through silica pad. The solvent was evaporated up to 100 mL and additional 100 mL methanol were added. Formed solid was filtered and recrystallized from toluene. Final purification was done by sublimation. Yield: 6.88 g (53.6 %)

**2-(3-(5-(9,9-dimethyl-9H-fluoren-4-yl)-3,6-diphenylpyrazin-2-yl)phenyl)-4,6-diphenylpyrimidine (E3)**

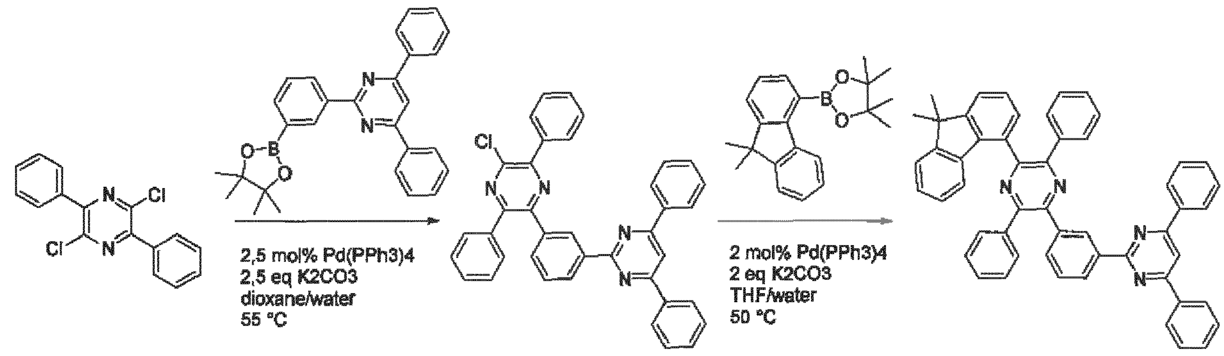


**1st Step. Synthesis of 2-(3-(5-chloro-3,6-diphenylpyrazin-2-yl)phenyl)-4,6-diphenylpyrimidine**

3-neck round-bottom flask was flushed with nitrogen and charged with 27.59 g 2,5-dichloro-3,6-diphenylpyrazine (CAS 74134-61-5) (1.05 eq), 37.90 g 4,6-diphenyl-2-(3-(4,4,5,5-tetramethyl-1,3,2-dioxaborolan-2-yl)phenyl)pyrimidine (CAS 1381862-91-4) (1 eq), 2.51 g tetrakis(triphenylphosphine)palladium(0) (CAS 14221-01-3) (0.025 eq), 30.15 g potassium carbonate (CAS 584-08-7) (2.5 eq). A deaerated mixture of 660 mL dioxane and 110 mL water was added. The reaction was running 2 days at 55 °C under nitrogen atmosphere. A yellow suspension formed; the solid precipitate was filtered and washed with water, dioxane and 2-methoxy-2-methylpropane. The raw product was extracted in Soxhlet apparatus with toluene. Final purification was done by sublimation. Yield: 20.63 g (41.27 %)

**2nd Step. Synthesis of 2-(3-(5-(9,9-dimethyl-9H-fluoren-4-yl)-3,6-diphenylpyrazin-2-yl)phenyl)-4,6-diphenylpyrimidine**

3-neck round-bottom flask was flushed with nitrogen and charged with 12.5 g 2-(3-(5-chloro-3,6-diphenylpyrazin-2-yl)phenyl)-4,6-diphenylpyrimidine (no CAS) (1 eq), 8.38 g 2-(9,9-dimethyl-9H-fluoren-4-yl)-4,4,5,5-tetramethyl-1,3,2-dioxaborolane (CAS 1365692-79-0) (1.2 eq), 0.504 g tetrakis-(triphenylphosphine)palladium(o) (CAS 14221-01-3) (0.02 eq), 6.03 g potassium carbonate (CAS 584-08-7) (2 eq). A deaerated mixture of 88 mL THF and 22 mL water was added. The reaction was running at 50 °C under nitrogen atmosphere overnight. Then the reaction mixture was cooled down, the solvents were evaporated and resulting solid was extracted in chloroform. The extract was washed with water, organic phase was filtered through silica pad and evaporated at low pressure. Further purification was done by recrystallization of the crude solid from toluene/acetonitrile 1:2 mixture and by sublimation. Yield: 12.70 g (82.65 %)

**4-(3-(5-(9,9-dimethyl-9H-fluoren-4-yl)-3,6-diphenylpyrazin-2-yl)phenyl)-2,6-diphenylpyrimidine ($E_4$)**

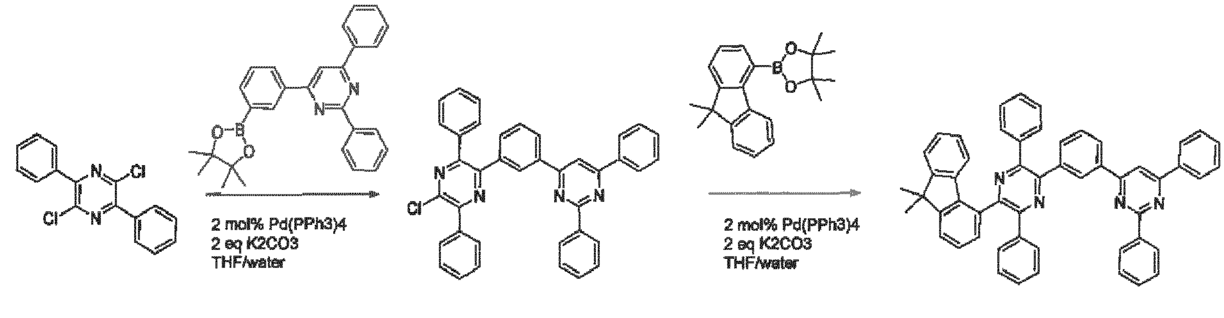

**1st Step. Synthesis of 4-(3-(5-chloro-3,6-diphenylpyrazin-2-yl)phenyl)-2,6-diphenylpyrimidine.**

3-neck round-bottom flask was flushed with nitrogen and charged with 22 g 2,5-dichloro-3,6-diphenylpyrazine (CAS 74134-61-5) (1.00 eq), 31.7 g 2,4-diphenyl-6-(3-(4,4,5,5-tetramethyl-1,3,2-dioxaborolan-2-yl)phenyl)pyrimidine (CAS 1342892-16-3) (1 eq), 1.69 g tetrakis(triphenylphosphine)palladium(o) (CAS 14221-01-3) (0.02 eq), 20.15 g potassium carbonate (CAS 584-08-7) (2.0 eq). A deaerated mixture of 300 mL THF and 75 mL water was added. The reaction was running 1 day at 55 °C under nitrogen atmosphere. A yellow suspension formed; the precipitate was filtered and washed with water, THF and acetonitrile, dissolved in chloroform and the solution filtered through silica pad. Further purification was done by sublimation. Yield: 17.3 g (38.6 %)

**2nd Step. Synthesis of 4-(3-(5-(9,9-dimethyl-9H-fluoren-4-yl)-3,6-diphenylpyrazin-2-yl)phenyl)-2,6-diphenylpyrimidine**

3-neck round-bottom flask was flushed with nitrogen and charged with 11.5 g 4-(3-(5-chloro-3,6-diphenylpyrazin-2-yl)phenyl)-2,6-diphenylpyrimidine (no CAS) (1 eq), 9.64 g 2-(9,9-dimethyl-9H-fluoren-4-yl)-4,4,5,5-tetramethyl-1,3,2-dioxaborolane (CAS 1365692-79-0) (1 eq), 0.45 g tetrakis-(triphenylphosphine)palladium(o) (CAS 14221-01-3) (0,02 eq), 5.52 g potassium carbonate (CAS 584-08-7) (2 eq). A deaerated mixture of 120 mL THF and 20 mL water was added. The reaction was running at 60 °C under nitrogen atmosphere for 2 days. Then the reaction mixture was cooled down, solvents were evaporated and the solid was extracted in chloroform, the extract washed with water. Organic phase was filtered through silica pad and evaporated at low pressure. Further purification was done by sequential recrystallization from toluene and chlorobenzene and final sublimation. Yield: 6.61 g (45.3 %).

**Synthesis of 2-(3-(5-(9,9-dimethyl-9H-fluoren-4-yl)-3,6-diphenylpyrazin-2-yl)phenyl)-4,6-diphenyl-1,3,5-triazme (E5)**

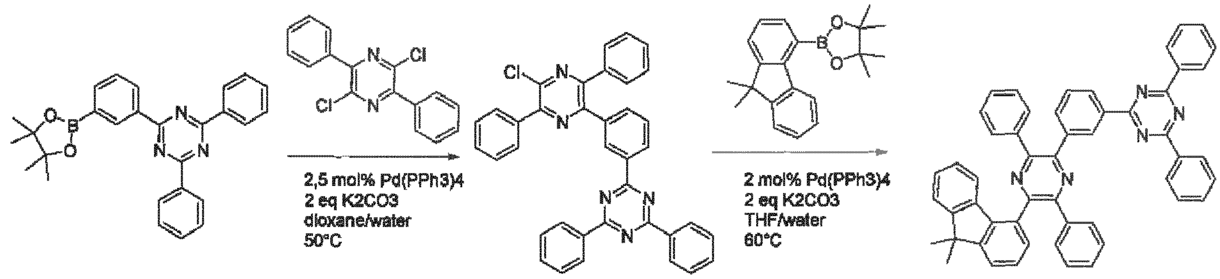


**1st Step. 2-(3-(4-chloro-3,6-diphenylpyrazin-2-yl)phenyl)-4,6-diphenyl-1,3,5-**triazine

3-neck round-bottom flask was flushed with nitrogen and charged with 40.0 g 2,4-diphenyl-6-(3-(4,4,5,5-tetramethyl-1,3,2-dioxaborolan-2-yl)phenyl)-1,3,5-triazine (CAS 1269508-31-7) (1 eq), 26.29 g 2,5-dichloro-3,6-diphenylpyrazine (CAS 74134-61-5) (0.95 eq), 2.65 g tetrakis(triphenylphosphine)palladium(o) (CAS 14221-01-3) (0.025 eq), 25.40 g potassium carbonate (CAS 584-08-7) (2 eq). A deaerated mixture of 368 mL dioxane and 92 mL water was added. Reaction was running 2 days at 50 °C under a nitrogen atmosphere. A yellow suspension formed. The reaction mixture was cooled down, the precipitate was filtered and washed with dioxane, water and methanol. The raw solid product was stirred in chlorobenzene at room temperature, filtered and the solid was discarded. The organic filtrate was further filtered through silica pad and evaporated. Final purification was done by sublimation. Yield: 22.8 g (44.8 %)

**2nd Step. 2-(3-(5-(9,9-dimethyl-9H-fluoren-4-yl)-3,6-diphenylpyrazin-2-yl)phenyl)-4,6-diphenyl-1,3,5-triazine.**

3-neck round-bottom flask was flushed with nitrogen and charged with 25 g 2-(3-(5-chloro-3,6-diphenylpyrazin-2-yl)phenyl)-4,6-diphenyl-1,3,5-triazine (no CAS) (1 eq), 18.1 g 2-(9,9-dimethyl-9H-fluoren-4-yl)-4,4,5,5-tetramethyl-1,3,2-dioxaborolane (CAS 1365692-79-0) (1.3 eq), 1.0 g tetrakis-(triphenylphosphine)palladium(o) (CAS 14221-01-3) (0.02 eq), 12.04 g potassium carbonate (CAS 584-08-7) (2 eq). A deaerated mixture of 160 mL THF and 40 mL water was added. The reaction was running at 60 °C under a nitrogen atmosphere for 3 days. A yellow suspension formed. The reaction mixture was cooled down, filtrated, washed with THF, water and methanol. The solid was dissolved in chloroform, filtered through silica pad. The solvent was evaporated at low pressure and the white crude solid was further purified by sublimation. Yield: 19.09 g (78 %).

**2-(3-(6-(9,9-dimethyl-9H-fluoren-4-yl)-3,5-diphenylpyrazin-2-yl)phenyl)-4,6-diphenylpyrimidine E6**

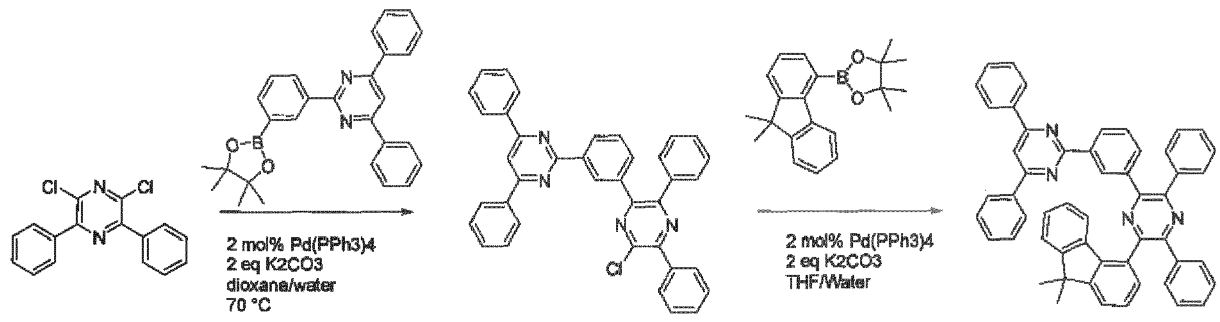


**1st Step. Synthesis of 2-(3-(6-chloro-3,5-diphenylpyrazin-2-yl)phenyl)-4,6-diphenylpyrimidine.**

3-neck round-bottom flask was flushed with nitrogen and charged with 22 g 2,6-dichloro-3,5-diphenylpyrazine (CAS 67714-55-0) (1.00 eq), 31.7 g 4,6-diphenyl-2-(3-(4,4,5,5-tetramethyl-1,3,2-dioxaborolan-2-yl)phenyl)pyrimidine (CAS 1381862-91-4) (1 eq), 1.69 g tetrakis (triphenylphosphine)palladium(o) (CAS 14221-01-3) (0.02 eq), 20.15 g potassium carbonate (CAS 584-08-7) (2.0 eq). A deaerated mixture of 280 mL dioxane and 70 mL water was added. The reaction was running 3 days at 70 °C under nitrogen atmosphere. A yellow suspension formed; the precipitate was filtered, the collected solid washed with water, dioxane and methanol and dissolved in chloroform. The solution was filtered through silica pad and evaporated. Further purification was done by recrystallization from toluene. Yield: 19.6 g (49 %)

**2nd Step. Synthesis of 2-(3-(6-(9,9-dimethyl-9H-fluoren-4-yl)-3,5-diphenylpyrazin-2-yl)phenyl)-4,6-diphenylpyrimidine.**

3-neck round-bottom flask was flushed with nitrogen and charged with 15 g 2-(3-(6-chloro-3,5-diphenylpyrazin-2-yl)phenyl)-4,6-diphenylpyrimidine (no CAS) (1 eq), 10.9 g 2-(9,9-dimethyl-9H-fluoren-4-yl)-4,4,5,5-tetramethyl-1,3,2-dioxaborolane (CAS 1365692-79-0) (1.3 eq), 0.61 g tetrakis-(triphenylphosphine)palladium(o) (CAS 14221-01-3) (0.02 eq), 7,23 gpotassium carbonate (CAS 584-08-7) (2 eq). A deaerated mixture of 160 mL THF and 25 mL water was added. The reaction was running at 60 °C under nitrogen atmosphere for 2 days. Then the reaction mixture was cooled down, precipitated solid was filtered, washed with water and extracted with chloroform. Organic phase was filtered through silica pad and evaporated at low pressure. Further purification was done by recrystallization from THF and final sublimation. Yield: 17 g (89 %).

Device experiments

*Comparative compounds C1 to C3*

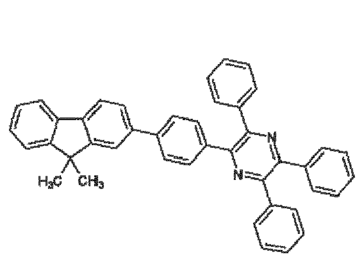

C1

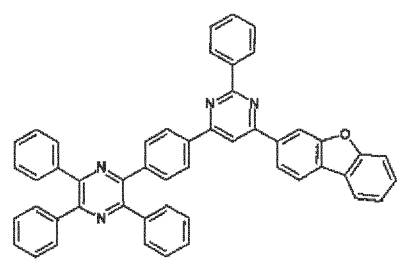

C2

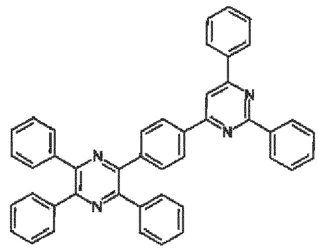

C3

Compound $C_1$ is known from WO2022/015047, compounds $C_2$ and $C_3$ from WO2019/238858.

*Support materials*

Supporting materials for device experiments

F1 is

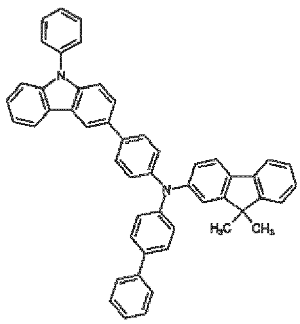

CAS 1242056-42-3

F2 is

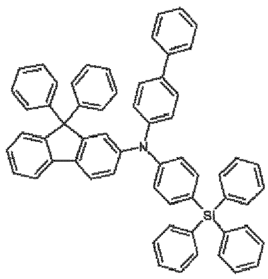

CAS 1613079-70-1

F3 is

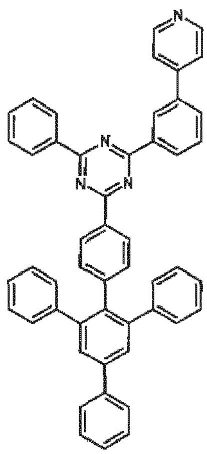

(published in EP 3 923 364)

PD2 is

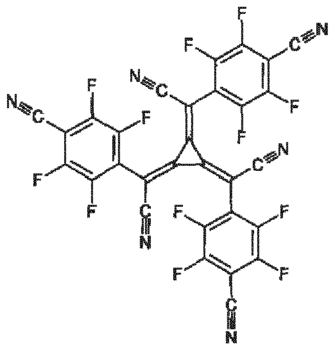

CAS 1224447-88-4

LiQ is lithium 8-hydroxyquinolinolate, CAS 850918-68-2.

H09 is an emitter host and BD200 is a blue fluorescent emitter dopant, both commercially available from SFC, Korea.

*Preparation of the model blue OLED device*

The device was made by depositing a 10 nm hole injection layer of F1 doped with PD2 (matrix to dopant weight ratio of 97.5:2.5 vol%) onto an ITO-glass substrate, followed by a 128 nm thick undoped hole transport layer of F1. A 5 nm layer electron blocking layer of F2 was deposited on the HTL. Subsequently, a blue fluorescent emitting layer of emitter host H09 (Sun Fine Chemicals) doped with BD200 (Sun Fine Chemicals) (97:3 vol%) was deposited with a thickness of 20 nm. On the emission layer, a 5 nm layer made of the tested compound or of the comparative compound was deposited as a hole blocking layer. A 31 nm layer electron transport layer made of F3 and LiQ was co-deposited in volume ratio 50:50 on the HBL. Subsequently, an aluminium layer with a thickness of 100 nm was deposited as a cathode. All depositions were made by vacuum thermal evaporation.

Table 1a schematically describes the model blue fluorescent bottom emission device.

Table 1a

| Layer | Material | c [vol%] | d [nm] |
|---|---|---|---|
| anode | ITO | 100 | 90 |
| HIL | F1:PD2 | 97.5:2.5 | 10 |
| HTL | F1 | 100 | 128 |
| EBL | F2 | 100 | 5 |
| EML | H09:BD200 | 97:3 | 20 |

(continued)

| Layer | Material | c [vol%] | d [nm] |
|---|---|---|---|
| HBL | Tested or comparative | 100 | 5 |
| ETL | F3:LiQ | 50:50 | 31 |
| cathode | Al | 100 | 100 |

ITO is indium tin oxide.

*Technical effect*

Surprisingly, it was found that the organic electronic device comprising organic semiconductor layer comprising compound of formula 1 according to the present invention solve the problem underlying by being superior over the organic electronic device known in the art, in particular with respect to operational voltage, current and quantum efficiency.

The results obtained with the above described model device are given in Table 1b

Table 1b

| HBL | CIE-y | U/V | Rel. V % | Corr. CEFF/CIE-y Cd/A | Rel. CEFF/CIE-y % | EQE [%] | Rel. EQE % |
|---|---|---|---|---|---|---|---|
| E1 | 0.098 | 3.83 | 94.1 | 105.0 | 112 | 11.41 | 112 |
| E2 | 0.097 | 3.72 | 91.4 | 104.8 | 112 | 11.36 | 112 |
| E3 | 0.097 | 3.73 | 91.6 | 105.3 | 113 | 11.44 | 113 |
| E4 | 0.097 | 3.82 | 93.9 | 107.6 | 115 | 11.68 | 115 |
| E5 | 0.097 | 3.87 | 95.1 | 98.2 | 105 | 10.64 | 105 |
| C1 | 0.098 | 4.07 | 100 | 93.4 | 100 | 10.16 | 100 |
| C2 | 0.098 | 4.83 | 119 | 58.7 | 63 | 6.39 | 63 |
| C3 | 0.098 | 4.42 | 109 | 70.6 | 76 | 7.68 | 76 |

In comparison with state-of-art compounds C1-C3, the inventive compounds E1 to E5 enable lower operational voltage and higher current and quantum efficiency.

The features disclosed in the foregoing description and in the dependent claims may, both separately and in any combination thereof, be material for realizing the aspects of the disclosure made in the independent claims, in diverse forms thereof.

## Claims

1. Compound of the following formula (I)

$$A\text{-}(Cy)_n\text{-}Pz\text{-}FL \qquad (I)$$

wherein

- A is selected from substituted or unsubstituted $C_{10}$ to $C_{42}$ aryl comprising at least two condensed aromatic rings and from $C_2$-$C_{42}$ heteroaryl, wherein it is excluded that the $C_2$ to $C_{42}$ heteroaryl is benzoxazolyl or carbazolyl;

  - wherein, if A is substituted, the substituent(s) is/are independently selected from the group consisting of $C_6$-$C_{18}$ aryl, $C_3$-$C_{20}$ heteroaryl, D, F, CN, $C_1$-$C_{20}$ alkyl, $C_1$-$C_{20}$ alkoxy, $PY(R)_2$, OR, SR, (C=O)R, (C=O)N(R)$_2$, $Si(R)_3$, (S=O)R, and

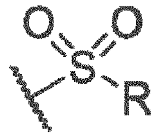,

whereby

Y is O or S, and
R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;

- Cy is independently selected from a substituted or unsubstituted monocyclic $C_3$ to $C_{36}$ hydrocarbon group, a substituted or unsubstituted polycyclic $C_3$ to $C_{36}$ hydrocarbon group, a substituted or unsubstituted monocyclic heteroatom-containing $C_3$ to $C_{36}$ hydrocarbon group, or a substituted or unsubstituted polycyclic heteroatom-containing $C_3$ to $C_{36}$ hydrocarbon group;

  - wherein, if Cy is substituted, the substituent(s) is/are independently selected from the groups consisting of $C_6$-$C_{18}$ aryl, $C_3$-$C_{20}$ heteroaryl, D, F, CN, $C_1$-$C_{16}$ alkyl, $C_1$-$C_{16}$ alkoxy, $PY(R)_2$, OR, SR, (C=O)R, $(C=O)N(R)_2$, $Si(R)_3$, (S=O)R, and

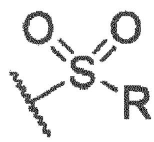,

whereby

Y is O or S, and
R is independently selected from $C_1$-$C_{20}$ linear alkyl, $C_1$-$C_{20}$ alkoxy, $C_1$-$C_{20}$ thioalkyl, $C_3$-$C_{20}$ branched alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl;

- n is an integer from o to 4;
- Pz is fully substituted pyrazinylene;

  - wherein the substituents on the pyrazinylene are independently selected from groups having at least one carbon atom;

- FL is substituted or unsubstituted fluorenyl;

  - wherein, if FL is substituted, the substituent(s) is/are independently selected from the groups consisting of $C_6$-$C_{18}$ aryl, $C_2$-$C_{20}$ heteroaryl, D, F, CN, $C_1$-$C_{16}$ alkyl, $C_1$-$C_{16}$ alkoxy, $PY(R)_2$, OR, SR, (C=O)R, $(C=O)N(R)_2$, $Si(R)_3$, (S=O)R, and

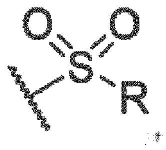

whereby

Y is O or S, and
R is independently selected from $C_1$-$C_{20}$ linear alkyl, $C_1$-$C_{20}$ alkoxy, $C_1$-$C_{20}$ thioalkyl, $C_3$-$C_{20}$ branched

alkyl, $C_3$-$C_{20}$ cyclic alkyl, $C_3$-$C_{20}$ branched alkoxy, $C_3$-$C_{20}$ cyclic alkoxy, $C_3$-$C_{20}$ branched thioalkyl, $C_3$-$C_{20}$ cyclic thioalkyl, $C_6$-$C_{20}$ aryl and $C_3$-$C_{20}$ heteroaryl.

**2.** Compound according to claim 1, wherein A comprises at least one six-membered N-containing aromatic ring.

**3.** Compound according to claim 1 or 2, wherein A is selected from the group consisting of aryl comprising 2, 3, 4 or 5 condensed aromatic rings, azinyl, diazinyl, triazinyl, azanaphtalenyl, diazanaphtalenyl, triazanaphtalenyl, azaanthracenyl, azaphenanthrenyl, diazaanthracenyl, diazaphenanthrenyl, triazaanthracenyl, triazaphenanthrenyl, benzofuranyl and aza-, diaza- or triaza- derivatives thereof, benzothiophenyl and aza-, diaza- or triaza- derivatives thereof, dibenzofuranyl and aza-, diaza- or triaza- derivatives thereof, dibenzothiophenyl and aza-, diaza- or triaza- derivatives thereof, naphtofuranyl and aza-, diaza- or triaza- derivatives thereof, naphtothiophenyl and aza-, diaza- or triaza- derivatives thereof, naphtobenzofuranyl and aza-, diaza- or triaza- derivatives thereof, naphtobenzothiophenyl and aza-, diaza- or triaza- derivatives thereof, dinaphtofuranyl and aza-, diaza- or triaza- derivatives thereof, and dinaphtothiophenyl and aza-, diaza- or triaza- derivatives thereof, which are substituted or unsubstituted, respectively.

**4.** Compound according to any of the preceding claims, wherein A is selected from naphtalenyl, anthracenyl, phenanthrenyl, triphenylenyl, acenaphtenyl, fluoranthenyl, pyrenyl, tetracenyl, pyridinyl, pyrazinyl, pyrimidinyl, pyridazinyl, triazinyl, quinolinyl, isoquinolinyl, cinnolinyl, phtalazinyl, quinazolinyl, quinoxalinyl, naphtyridinyl, imidazopyridinyl, phenathridinyl, phenanthrolinyl, acridinyl, benzoacridinyl, dibenzoacridinyl, which are substituted or unsubstituted, respectively.

**5.** Compound according to any of the preceding claims, wherein if A is substituted, the substituent(s) is/are independently selected from $C_6$-$C_{12}$ aryl.

**6.** Compound according to any of the preceding claims, wherein Cy is independently selected from the group consisting of $C_6$-$C_{36}$ aryl and $C_3$-$C_{36}$ heteroaryl.

**7.** Compound according to any of the preceding claims, wherein Cy is monocyclic or bicyclic, respectively.

**8.** Compound according to any of the preceding claims, wherein the substituents of the fully substituted pyrazinylene are independently selected from a hydrocarbyl-group, a carbocyclic group, or a heterocyclic group.

**9.** Compound according to any of the preceding claims, wherein the substituents of the fully substituted pyrazinylene are independently selected from the group consisting of $C_6$-$C_{36}$ aryl and $C_3$-$C_{36}$ heteroaryl.

**10.** Compound according to any of the preceding claims, wherein the substituents of the fully substituted pyrazinylene are monocyclic or bicyclic.

**11.** Compound according to any of the preceding claims, wherein the fluorenyl is substituted at the 9-position thereof with two hydrocarbyl groups which may be linked with each other to form a ring.

**12.** Compound according to any of the preceding claims, wherein the fluorenyl is 9,9'-dimethyl-fluorenyl.

**13.** Organic semiconducting material comprising the compound according to any of the preceding claims.

**14.** Organic electronic device comprising a semiconductor layer, wherein the semiconductor layer comprises the compound according to any of the claims 1 to 12.

**15.** Organic electronic device according to claim 14, wherein the semiconducting layer is an electron transporting layer.

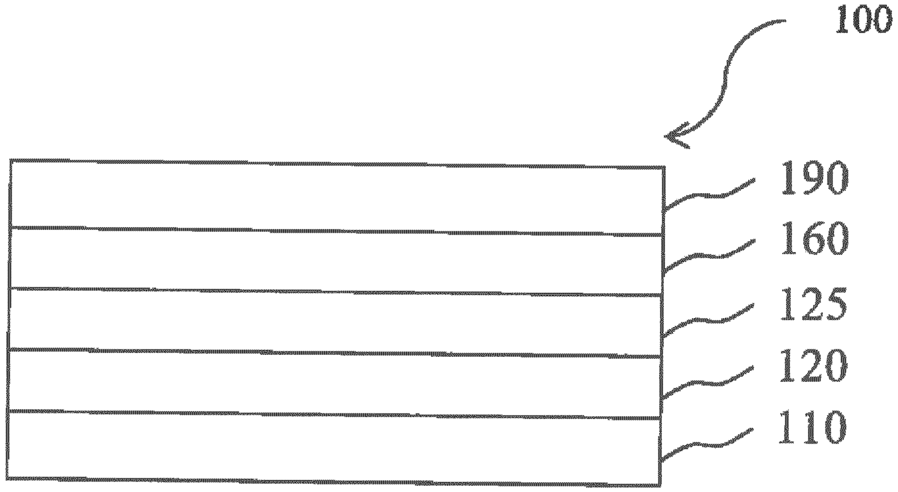


Fig.1

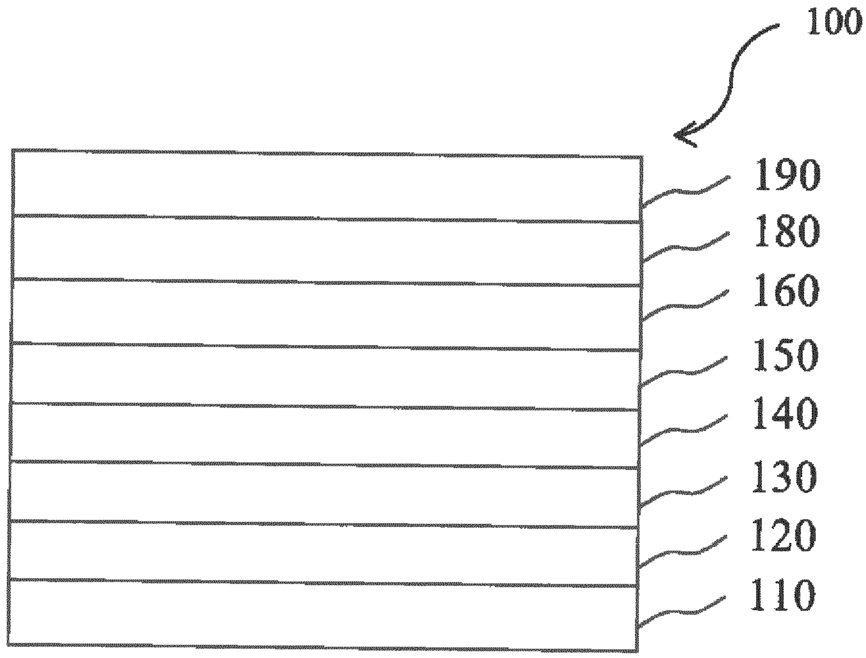


Fig.2

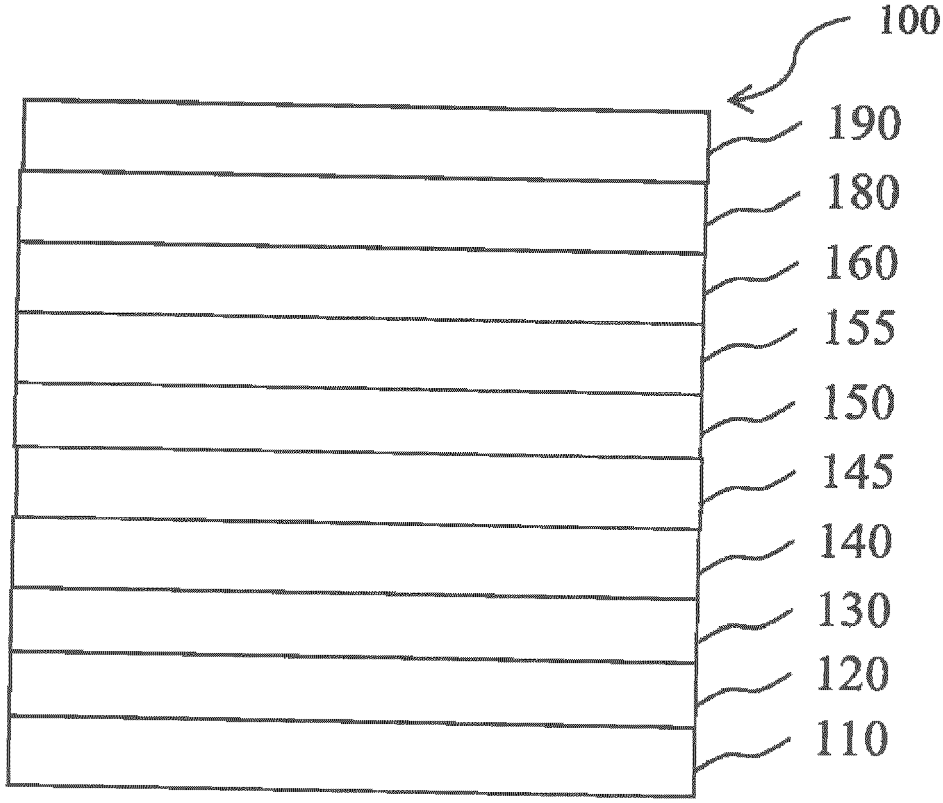


Fig.3

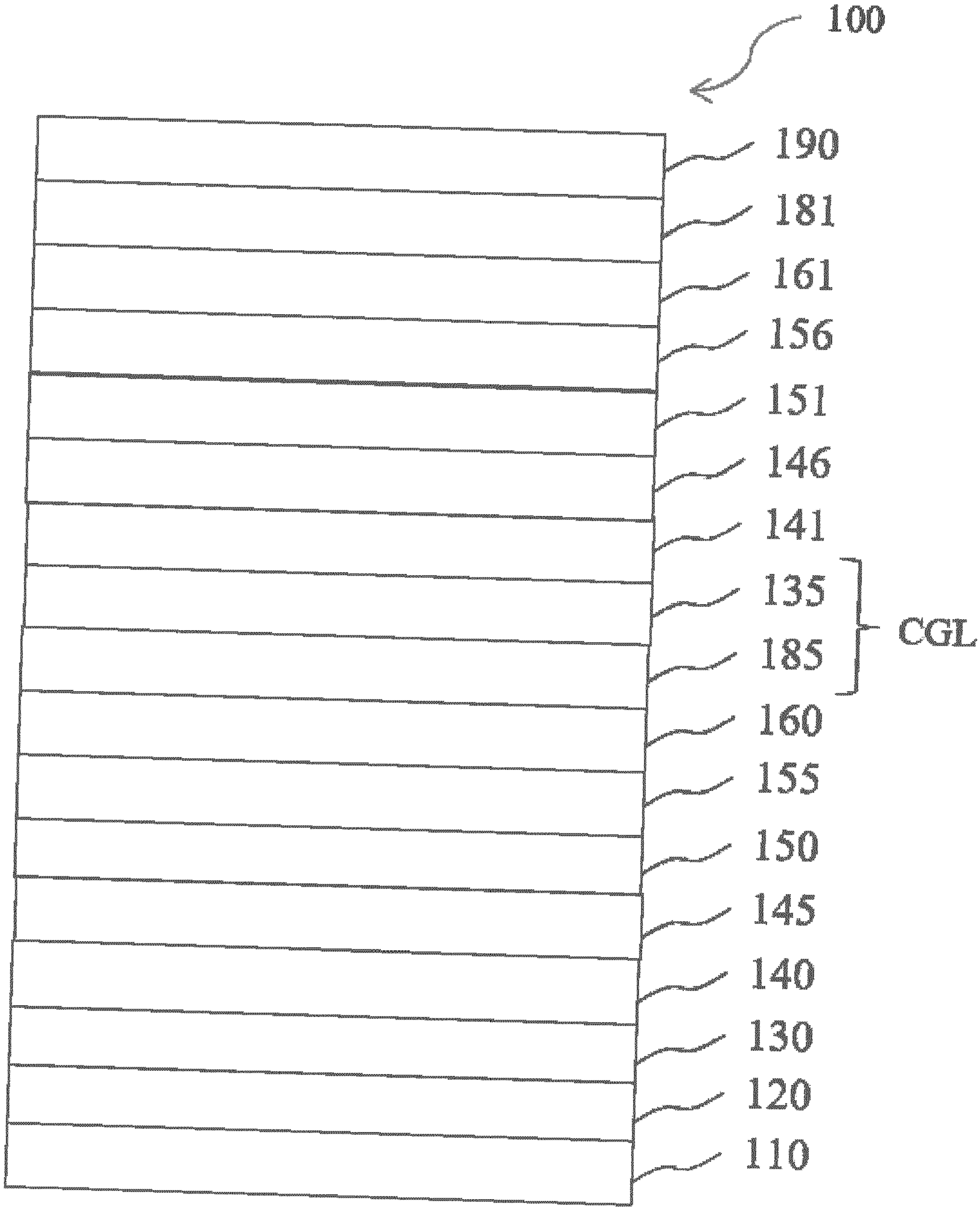
100
190
181
161
156
151
146
141
135
185
CGL
160
155
150
145
140
130
120
110

Fig.4

Europäisches Patentamt
European Patent Office
Office européen des brevets

# EUROPEAN SEARCH REPORT

Application Number
EP 22 18 2147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2022/015047 A1 (SOLUS ADVANCED MAT CO LTD [KR]) 20 January 2022 (2022-01-20)<br>* claims 1, 12 *<br>* page 64; compounds A2-A4, A6 *<br>* page 29, paragraph 131 *<br>----- | 1-15 | INV.<br>C07D403/10<br>H10K85/00 |
| X | CN 110 229 145 A (JIANGSU SUNERA TECH CO LTD) 13 September 2019 (2019-09-13)<br>* claims 1, 9 *<br>* page 10; compound 175 *<br>* page 9; compound 160 *<br>----- | 1-15 | |
| X | EP 2 489 664 A1 (IDEMITSU KOSAN CO [JP]) 22 August 2012 (2012-08-22)<br>* claims 1, 21 *<br>* page 21, 2-nd row, 4-th compound *<br>----- | 1,3-15 | |
| A | JP 2010 040830 A (KONICA MINOLTA HOLDINGS INC) 18 February 2010 (2010-02-18)<br>* page 141; compound HC9 *<br>----- | 1 | TECHNICAL FIELDS SEARCHED (IPC)<br>C07D<br>H01L |

The present search report has been drawn up for all claims

3

| Place of search | Date of completion of the search | Examiner |
|---|---|---|
| Munich | 13 January 2023 | Gutke, Hans-Jürgen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 22 18 2147

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

**see sheet B**

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 22 18 2147

The Search Division considers that the present European patent application does not comply with the requirements of unity of invention and relates to several inventions or groups of inventions, namely:

1. claims: 1, 3-15(all partially)

   compounds of formula I wherein the group -(Cy)n-A comprises carbocyclic aromatic rings

   ---

2. claims: 2(completely); 1, 3-15(partially)

   compounds of formula I wherein the group -(Cy)n-A comprises at least one heterocyclic aromatic ring

   ---

## ANNEX TO THE EUROPEAN SEARCH REPORT ON EUROPEAN PATENT APPLICATION NO. EP 22 18 2147

This annex lists the patent family members relating to the patent documents cited in the above-mentioned European search report. The members are as contained in the European Patent Office EDP file on
The European Patent Office is in no way liable for these particulars which are merely given for the purpose of information.

13-01-2023

| Patent document cited in search report | Publication date | Patent family member(s) | Publication date |
|---|---|---|---|
| WO 2022015047 A1 | 20-01-2022 | KR 20220008636 A | 21-01-2022 |
| | | WO 2022015047 A1 | 20-01-2022 |
| CN 110229145 A | 13-09-2019 | CN 110229145 A | 13-09-2019 |
| | | CN 112851653 A | 28-05-2021 |
| EP 2489664 A1 | 22-08-2012 | CN 102471320 A | 23-05-2012 |
| | | EP 2489664 A1 | 22-08-2012 |
| | | JP WO2011046182 A1 | 07-03-2013 |
| | | KR 20120052231 A | 23-05-2012 |
| | | TW 201118150 A | 01-06-2011 |
| | | US 2012126217 A1 | 24-05-2012 |
| | | WO 2011046182 A1 | 21-04-2011 |
| JP 2010040830 A | 18-02-2010 | JP 5338184 B2 | 13-11-2013 |
| | | JP 2010040830 A | 18-02-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005086251 A
- EP 1837926 B1
- WO 2007107306 A
- WO 2007107356 A
- EP 2722908 A1
- EP 1970371 A1
- WO 2013079217 A1
- WO 2022015047 A
- WO 2019238858 A


### Non-patent literature cited in the description


- **YASUHIKO SHIROTA ; HIROSHI KAGEYAMA.** *Chem. Rev.,* 2007, vol. 107, 953-1010
- *CHEMICAL ABSTRACTS,* 74134-61-5
- *CHEMICAL ABSTRACTS,* 333432-28-3
- *CHEMICAL ABSTRACTS,* 14221-01-3
- *CHEMICAL ABSTRACTS,* 584-08-7
- *CHEMICAL ABSTRACTS,* 1381862-91-4
- *CHEMICAL ABSTRACTS,* 57038-62-7
- *CHEMICAL ABSTRACTS,* 72287-26-4
- *CHEMICAL ABSTRACTS,* 1365692-79-0
- *CHEMICAL ABSTRACTS,* 1342892-16-3